# EUROPEAN PATENT SPECIFICATION

(11) **EP 3 558 385 B1**
(45) Date of publication and mention of the grant of the patent: **11.08.2021**
(21) Application number: 17838172.9
(22) Date of filing: 19.12.2017
(51) Int. Cl.: A61K 47/61, A61K 47/69, A61P 17/02

(54) **METHOD OF PREPARATION OF A MEDICAL PREPARATION WITH A CARRIER BASED ON WATER INSOLUBLE HYALURONAN CONJUGATED TO AMINO ACIDS OR PEPTIDES**
VERFAHREN ZUR HERSTELLUNG EINES MEDIZINISCHEN PRÄPARATS MIT EINEM TRÄGER AUF BASIS VON WASSERUNLÖSLICHEN HYALURONAN, DAS AN AMINOSÄUREN ODER PEPTIDE KONJUGIERT IST
PROCÉDÉ DE PRÉPARATION D'UNE PRÉPARATION MÉDICALE COMPRENANT UN SUPPORT À BASE D'HYALURONANE INSOLUBLE DANS L'EAU CONJUGUÉ À DES ACIDES AMINÉS OU DES PEPTIDES

(30) Priority: 22.12.2016 CZ 20160826
(43) Date of publication of application: 30.10.2019
(73) Proprietor: Contipro A.S., 561 02 Dolní Dobrouc (CZ)
(72) Inventor: KAREL, Sergej, 561 51 Letohrad (CZ); FLEGEL, Martin, 25244 Psary - Dolni Jircany (CZ); SULAKOVA, Romana, 56201 Usti Nad Orlici (CZ); FRYCAK, Roman, 78314 Bohunovice (CZ); SOGORKOVA, Jana, 33814 Hradek U Rokycan (CZ); BETAK, Jiri, 56961 Dolni Ujezd (CZ); CHMELAR, Josef, 75501 Vsetin (CZ); ZAPOTOCKY, Vojtech, 27401 Slany (CZ); VELEBNY, Vladimir, 56401 Zamberk (CZ)
(74) Representative: Lunzarová, Lucie
(86) International application number: PCT/CZ2017/050061
(87) International publication number: WO 2018/113802

(56) References cited:
- EP-A1- 2 360 188
- WO-A1-89/02445
- WO-A1-94/02517
- WO-A1-2011/097342
- GLASS J R ET AL: "Characterization of a hyaluronic acid--Arg--Gly--Asp peptide cell attachment matrix", BIOMATERIALS, ELSEVIER SCIENCE PUBLISHERS BV., BARKING, GB, vol. 17, no. 11, 1 January 1996 (1996-01-01), pages 1101-1108, XP004032670, ISSN: 0142-9612, DOI: 10.1016/0142-9612(96)85911-4
- Anonymous: "Speciality Hyaluronan Chemicals Product catalog", , 1 October 2015 (2015-10-01), pages 1-52, XP055462986, Retrieved from the Internet: URL:https://contipro.cz/images/documents/S peciality-Hyaluronan-Chemicals.pdf [retrieved on 2018-03-27]
- SEDOVÁ PETRA ET AL: "Preparation of hyaluronan polyaldehyde-a precursor of biopolymer conjugates", CARBOHYDRATE RESEARCH, PERGAMON, GB, vol. 371, 8 February 2013 (2013-02-08), pages 8-15, XP028527233, ISSN: 0008-6215, DOI: 10.1016/J.CARRES.2013.01.025
- CAROLE E SCHANT ET AL: "Chemical modifications of hyaluronic acid for the synthesis of derivatives for a broad range of biomedical applications", CARBOHYDRATE POLYMERS, APPLIED SCIENCE PUBLISHERS, LTD. BARKING, GB, vol. 85, no. 3, 11 March 2011 (2011-03-11) , pages 469-489, XP028383766, ISSN: 0144-8617, DOI: 10.1016/J.CARBPOL.2011.03.019 [retrieved on 2011-03-21]

## Description

### Field of the Invention

The invention relates to a method of preparation of a medical preparation with a carrier based on hyaluronan and/or its derivatives where the carrier used directly carries the active agent and is present in various forms.

### Background of the Invention

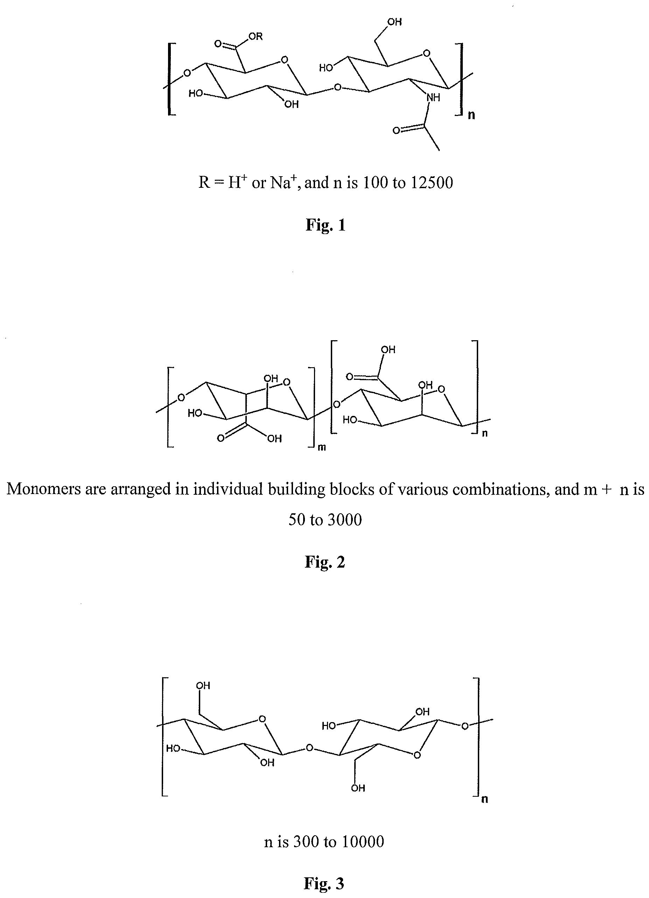

Hyaluronic acid (hyaluronan, HA) is known as a non-sulphated glycosaminoglycan composed of two repeating units of D-glucuronic acid and *N*-acetyl-D-glucosamine, linked via β(1→4) and β(1→3) glycosidic bonds, see Fig. 1. The molecular weight of the native hyaluronic acid is in the range of 5.10⁴ to 5.10⁶ g.mol⁻¹. This significantly hydrophilic polysaccharide forms part of connective tissues, skin, synovial fluid of joints, and plays an important role in many biological processes such as organization of proteoglycans, hydration, and cells differentiation. In view of the fact that this polymer is biocompatible, inherent to the body, and degradable, it becomes a suitable substrate for tissue engineering or as a carrier of bioactive substances.¹

The use of hyaluronan and its derivatives with modified or improved properties in the field of medicine is quite wide. Chemical modification of hyaluronan is usually preformed in two reaction centres: on the carboxyl group and on the primary hydroxyl group. An amino group can also be used after cleavage of N-acetyl group. It is not clear which of the hydroxyl group of the saccharide unit participates in the reaction; many results indicate the preference of the primary hydroxyl (C6) of the N-acetylglucosamine unit because of the highest reactivity of this group.

The carboxyl group can be converted into an amid or ester. Activating agents conventionally used for chemical preparation of peptides, such as carbodiimides²⁻⁵ or other agents, such as 2-chloro-1-methylpyridiniumiodide⁶, 2-chloro-dimethoxy-1,3,5-triazine⁷, carbonyldiimidazole⁸ etc., can be used for the conversion of the carboxy group to an amide. After increasing the carboxyl reactivity due its conversion into a more reactive intermediate, an amine is added to provide an amide by the condensation reaction. An ester can be formed by an alkylation reaction with alkylhalogenides^{9,10} methyl ester synthesis by a reaction with diazomethane^{11,12}, using of epoxides¹³⁻¹⁶ etc.

The modification of a hydroxyl group leads to the formation of an ether or ester bond. Ether can be easily prepared by the reaction with epoxides¹⁷⁻²⁰, divinylsulphone²¹⁻²³, ethylensulphide²⁴, or by formation of hemiacetal with the use of glutardehyde^{25,26}. Symmetric or mixed anhydrides²⁷⁻²⁹ can be used for an ester synthesis, or *O*-acyl-*O'*-alkylcarbonate³⁰ is used as an activating agent. These structural modifications lead to a crosslinking reaction to form hydro gels.

Solid Phase Peptide Synthesis, SPPS, is a method where the individual amino groups are gradually linked to the polymer carrier. Protecting groups (permanent or temporary) are used for all amino acids to decrease the risk of side reactions and formation of undesirable sequences. Protecting groups such as Fmoc and Boc are the most frequently used, where Fmoc protecting group can be cleaved in a basic environment and Boc protecting group can be cleaved in an acid environment. Solid phase peptide synthesis comprises repeating of the cycles of "condensation - washing - cleavage of protecting groups - washing". The synthetized peptide remains anchored to the polymer carrier until its desired sequence is obtained. Then the cleavage from the carrier and purification are performed.

Nowadays there is an effort to use the carriers that can be biocompatible and optionally biodegradable. The carries based on polysaccharides from alginate, agar, chitin, hyaluronan or cotton have been described. So far, the peptide has been always cleaved from the carrier. Its beneficial biological properties, that can mutually positively influence the biological properties of the peptide^{31,32} synthetized or anchored on the carrier, have not been used.

Alginates are the most common carriers used for the reaction of peptides and amino acids with polysaccharides. Alginate is a linear negatively charged polysaccharide consisting of repeating monomer units of β-D-mannuric acid and α-L-guluronic acid linked via (1→4) *O-*glykosidic bonds, as it is shown in the structural formula of alginate in Fig. 2.

The use of alginate as a drug form for a pharmaceuticals was described; however the processes were most often used for non-covalent anchoring of these drugs in a matrix or a gel. The alginate gel has been used also for analytical procedures of capturing the high molecular peptides, mainly enzymes, cell organelles, or the whole cells.^{33,34}

Covalent bonding of peptides and drugs enables their use as sophisticated dosage systems enhancing pharmacokinetics and bioavailability which enhances the clinical potential of a drug. The conjugate alginate-peptide is formed by an amidic bond between the carboxyl group of the alginate and the amino group of the peptide.³³⁻³⁵

The alginates with covalently bound peptides can be used in treatment of an injury. Wound healing is a tissue reaction to its injury. It is a complicated biological process comprising chemotaxis, cell proliferation, production of extracellular proteins, neovascularization etc. One of the possibilities of the wound healing treatment is influencing the healing process by natural stimulation agents such as growth factor.³⁵

Materials based on alginates have been prepared, which were modified by a peptide, and their effectivity in the wound healing process was studied on the injured skin *in vivo.* Rabbits treated with the alginate bandage containing the hybrid peptide Ser-Ile-Arg-Val-X-Val-X-Pro-Gly (where X = Ala or Gly) showed significantly higher epithelization and larger volume of regenerated tissue in comparison with other peptides Ser-Ile-Lys-Val-Ala-Val and Val-Pro-Val-Ala-Pro-Gly that were also anchored on the alginate bandage.³⁵

Cellulose in the form of paper was also used for the preparation of the peptides. From the chemical point of view, cellulose is a linear polysaccharide consisting of repetitive monomer units of d-glucose linked via β(1→4) *O*-glycosidic bonds, according to the structural formula in Fig. 3. Peptides can be obtained with low yield only which is not desirable. Paper has also the disadvantage of low mechanical stability.^{31,36}

Cotton is the purest form of cellulose, except of microbial cellulose, and it can be obtained in various forms and shapes; the possibility of the use of cotton as the carrier for SPPS has been studied. In this case, the anchoring of the peptide was performed by means of an ester bond between the hydroxyl group of cotton and the carboxyl group of the peptide. The synthesis was most often performed in DIC/HOBt/DMAP in DMF.^{31,37,38}

Nowadays, carriers based on hyaluronan in the form of fibres³⁹⁻⁴², thin films^{43,44}, or nonwoven fabrics⁴⁰ are available. The use of these perspective forms could remove some of the described drawbacks of the polysaccharide carriers used so far. There is still the lack of suitable carriers of pharmaceutical drugs.

The new method described in this invention has the advantage of the use of a carrier based on hyaluronan in the form of fibres, thin films, or nonwoven fabrics for solid-phase synthesis.

This carrier does not change during the reaction, i.e. it remains in the form of fibres, thin films, or nonwoven fabrics. Thus, it is a solid-phase synthesis where the peptide is constructed by individual amino acids or it is linked, as a whole, to the hyaluronan material. So far, hyaluronan has been used for the modification in solution, i.e. hyaluronan was dissolved and the reaction performed. Then the derivative was used for the preparation of the material. The difference between liquid-phase and solid-phase synthesis is that in case of solution synthesis and subsequent spinning, the peptide is also present inside the fibre, so it is less available. The solid-phase synthesis not only saves the costs, when the peptide is on the surface only, but spares from the optimization of the spinning processes which are different for individual derivatives.

Glass J R et al.: « Characterization of a hyaluronic acid --Arg--Gly--Asp peptide cell attachment matrix », Biomaterials, Elsevier Science Publishers BV., Barking, GB, vol. 17, no. 11, 1 January 1996, pages 1101-1108 disclose a method where hyaluronic acid is first made insoluble in water by crosslinking, then the resulting crosslinked hyaluronic acid is oxidated to form reactive aldehydes, and then the peptide is coupled thereto. In this method, the oxidation used results in opening the D-glucuronic cycles of hyaluronic acid.

Sedová Petra et al.: "Preparation of hyaluronan polyaldehyde-a precursor of biopolymer conjugates", Carbohydrate Research, Pergamon, GB, vol. 371, 8 February 2013, pages 8-15, discloses a method which proceeds in a solution.

### Summary of the Invention

The invention is defined by the claims. Any subject-matter falling outside the scope of the claims is provided for information purposes only.

The abovementioned drawbacks are solved by the method of preparation of the medical preparation with a carrier based on hyaluronan and/or derivatives thereof defined in the appended claims.

The present invention relates to a method of preparation of a medical preparation based on hyaluronan and/or palmitoyl hyaluronan and/or formyl hyaluronan according to the general formula

**A-S-N** **(X),**

which is represented by the formula: 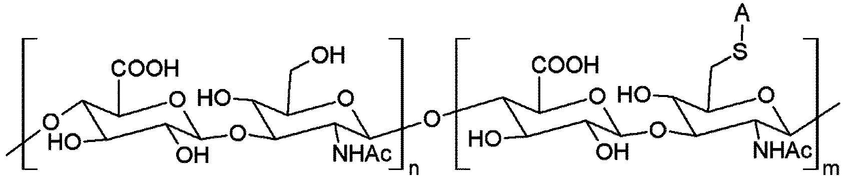 where
A is the medical substance selected from the group consisting of amino acids and peptides,
S is -O-C(=O)-, -NH-, or a linear linker which is a peptide formed by hydrophobic amino acids Xaa,
N is the carrier being hyaluronic acid and/or palmitoyl hyaluronan and/or formyl hyaluronan, which is in the form of an endless fibre, thread, fabrics, thin film, staple fibre and/or nonwoven textile,
m is 10 to 1250,
n is 100 to 12500, as defined in the claims.

The medical preparation prepared by the method according to the invention is characterized in that it is water-insoluble and it is biodegradable.

The medical preparation prepared by the method according to the invention is further characterized in that the carrier enables to prepare the conjugate bonded thereto, without the carrier being dissolved. In other words, the carrier remains insoluble during the whole preparation process, i.e. in solid phase, wherein at the same time it maintains its biodegradability.

The medical preparation prepared by the method according to the invention is further characterized in that the medical substance is bonded onto the carrier in the position 6 of the glucosamine part of the conjugate of hyaluronic acid and its derivatives of the general formula **X.**

According to the preparation method of the invention, the medical substance is anchored to hyaluronic acid and/or palmitoyl hyaluronan and/or formyl hyaluronan via (a) an ester bond (Scheme 1) or (b) by reductive amination (Scheme 2).

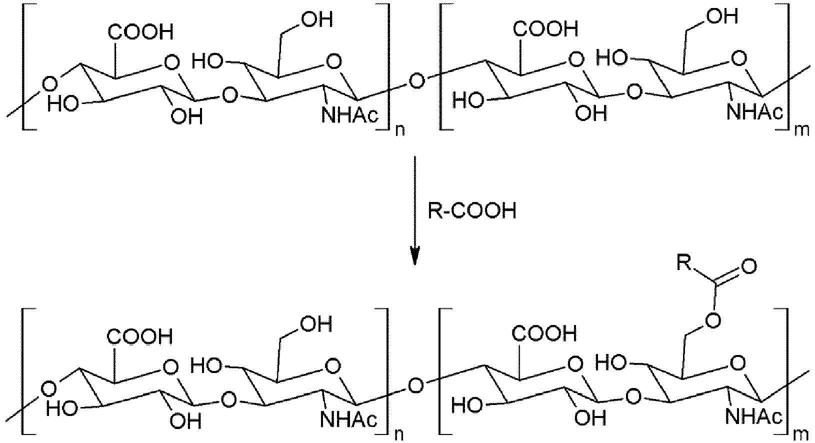

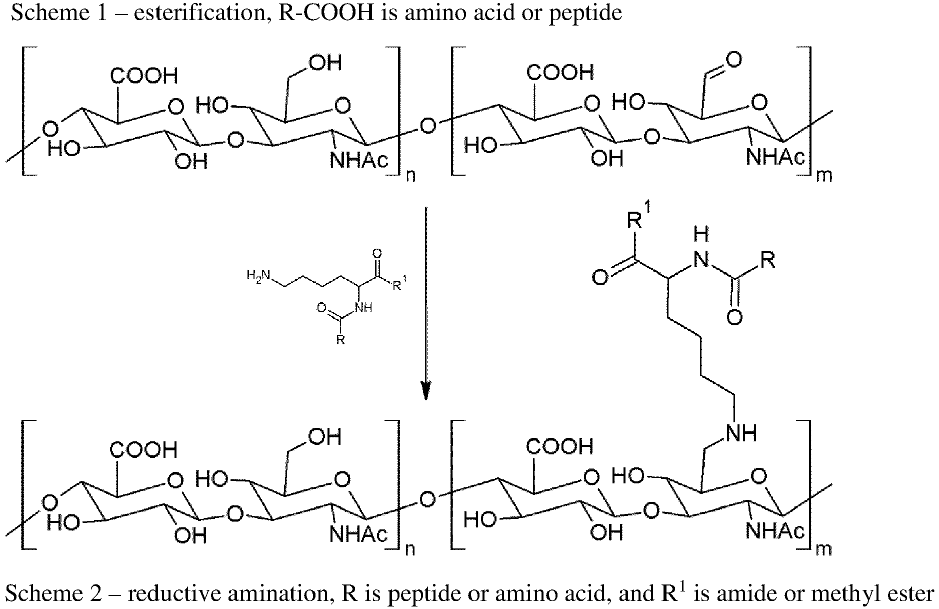

The carrier N is hyaluronic acid, palmitoyl hyaluronan, or formyl hyaluronan, wherein the carrier has the molecular weight in the range of 1.5x10⁴ to 2.5x10⁶ g.mol⁻¹.

The subject of the invention is a method of preparation of the medical preparation with a carrier based on hyaluronan and/or palmitoyl hyaluronan and/or formyl hyaluronan characterized in that the carboxyl group of the amino acid or peptide in the medical substance A, where the medical substance also comprises an N-terminal protecting group, is activated with a condensation agent in an aprotic polar solvent to form a reactive intermediate I, which reacts with the carrier in the presence of an organic base and a catalyst to form a conjugate of hyaluronic acid and/or palmitoyl hyaluronan and/or formyl hyaluronan with the medical substance. Thereafter, the terminal N-protecting group of the medical substance is cleaved off in a basic medium, preferably selected from the group of 20% piperidine in *N*,*N*-dimethylformamide, 2% 1,8-diazabicyclo[5.4.0]undec-7-ene in *N,N-*dimethylformamide, 30% tert-butylamine in *N,N-*dimethylformamide, preferably in 20% piperidine in *N*,*N*-dimethylformamide.

The medical substance A is an amino acid or a peptide, and it is linked to the carrier directly, or via a linear linker which is a peptide formed by hydrophobic amino acids Xaa, preferably Xaa-Ahx-Ahx-Xaa.

The method according to the invention is characterized in that the formation of the conjugate of hyaluronic acid and its derivatives is preferably performed at the temperature of 20°C to 40°C, preferably at the temperature of 22°C to 25°C, for 2 to 48 hours, preferably for 24 hours. The method according to the invention is further characterized in that the condensation agent used is preferably selected from the group of 4-nitrophenol, *N*-hydroxy succinimide, 2,4,5-trichlorophenol, 2,3,4,5,6-pentafluorophenol, 2,3,4,5,6-pentachlorophenol, *N*,*N'*-diisopropyl carbodiimide, *N,N'*-dicyclohexyl carbodiimide,1-[bis(dimethylamino) methylen]-1*H*-1,2,3-triazolo[4,5-b] pyridinium3-oxide hexafluoro phosphate, 1-ethyl-3-(3-dimethylaminopropyl) carbodiimide, ethylchloroformiate, 2-(1*H*-benzotriazole-1-yl)-1,1,3,3-tetramethyl uronium hexafluoro phosphate, benzotriazol-1-yl-oxy-tris(dimethylamino)-phosphonium hexafluoro phosphate, *O*-(benzotriazol-1-yl)-*N,N,N',N'*-tetramethyl uronium tetrafluoro borate, 4-triethylamino-dicyclohexyl karbodiimide *p*-toluensulphonate, propyl phosphonic acid anhydride, preferably *N*,*N'*-diisopropyl carbodiimide.

The method according to the invention is further characterized in that the polar aprotic solvent used is preferably selected from the group consisting of *N,N*-dimethyl formamide, dimethyl sulphoxide, *N*-methyl-2-pyrrolidone, acetonitrile, dichloromethane, tetrahydrofuran, 1,4-dioxane, preferably *N,N*-dimethyl formamide.

The method according to the invention is further characterized in that the organic base used is preferably selected from the group consisting of triethylamine, pyridine, morpholine, *N*-methyl morpholine, *N*,*N'*-diisopropyl ethylamine, imidazole.

The method according to the invention is also characterized in that the used catalyst is preferably selected from the group consisting of ethyl (hydroxyimino) cyanoacetate, hydroxyl benzo triazole, *N,N*-dimethyl amino pyridine or 1-hydroxy-7-azabenzo triazole, preferably the combinations of ethyl (hydroxyimino) cyanoacetate and *N,N-* dimethyl amino pyridine.

The method according to the invention is preferably also characterized in that the amount of the amino acid or peptide used, i.e. the medical substance, corresponds to 1 to 5 equivalents, preferably 3 equivalents, based on the dimer of hyaluronic acid and/or palmitoyl hyaluronan and/or formyl hyaluronan, the amount of the activating (condensation) agent used corresponds to 0.1 to 5 equivalents, preferably 3 equivalents, based on the dimer of hyaluronic acid and/or palmitoyl hyaluronan and/or formyl hyaluronan, the amount of the base used corresponds up to 10 equivalents, based on the dimer of hyaluronic acid and/or palmitoyl hyaluronan and/or formyl hyaluronan, and the amount of the catalyst used corresponds 0.1 to 5 equivalents, preferably 3 equivalents of ethyl (hydroxyimino) cyanoacetate and 0.3 equivalents of *N,N*-dimethyl amino pyridine, per dimer of hyaluronic acid and/or palmitoyl hyaluronan and/or formyl hyaluronan. In the preferred embodiment of the invention, the molar ratio of the medical substance, condensation agent, catalyst, and hyaluronic acid dimer is 1:1:1:1.

The medical preparation in the form of the conjugate prepared by the method according to the invention is for medical use as a dosage form of an active peptide or amino acid, intended for dermal, sublingual, oral, buccal, or local administration into an open wound.

The medical preparation prepared by the method according to the invention for dermal administration can contain for example the peptide Dalargin as the medical substance. The medical preparation prepared by the method according to the invention for buccal or sublingual administration can contain for example the peptide selected from the group of Desmopressin, Lysipressin or Glypressin, as the medical substance. The medical preparation prepared by the method according to the invention for buccal administration can contain for example the peptide selected from the group of antiviral drugs and adjuvants, or releasing factor for luteinizing and follicle-stimulating hormone. The medical preparation prepared by the method according to the invention for the direct administration to an open wound can contain, as the medical substance, for example the peptide selected from the group of Glypressin, Dalargin or AdDP as an immunostimulant.

Thus, the subject of the invention is the method of preparation of compounds of the general formula

**A-S-N** **(X),**

where the solid carrier N is made of hyaluronan and/or palmitoyl hyaluronan and/or formyl hyaluronan in the form of an endless fibre, thread, fabric, thin film, staple fibre and nonwoven fabric comprising the medical substance A linked by means of the linkage S, as defined in the claims. The method of the preparation of this complex system by the procedure in solid phase or by bonding the medical substance into a complex system is described in the examples below.

The method of linking the medical substance with the carrier is performed by an ester bond or by reductive amination. This biocompatible and biodegradable solid carrier is characterized in that it does not need to be cleaved from the medical substance during its various, mainly medical, activities, and it can preferably further act as modern non-invasive dosage form facilitating the penetration of the biologically active peptides or amino acids bound on said biocompatible carrier through mucosa and skin, and thus it serves as a complex and suitable material used for medical applications in human and veterinary medicine. The medical preparation prepared by the method according to the invention acts as a whole during the biological activity and the medical substance can be released slowly, in a prolonged manner from the carrier, which is subsequently eliminated naturally and intrinsically. The carrier can also affect the total effect of the medical substance bonded thereon and facilitate its use.

Table 1 presents peptides and their medical effect and the method of their administration. It shows that the medical preparation prepared by the method according to the invention for dermal administration comprises peptide Dalagrin as the medical substance having a healing effect, or Oxytocin having hormonal effect. For oral, buccal or sublingual administration, the medical preparation prepared by the method according to the invention comprises the peptide Desmopressin showing an antidiuretic effect, influence on blood coagulation by increasing the VIII factor, Lysipressin having presoric effect mainly in dental surgery, or Glypressin (Terlipressin) with a prolonged pressoric effect suitable to stop bleeding. For buccal administration, the medical preparation prepared by the method according to the invention comprises a peptide as the medical substance, used as antiviral agent and adjuvant, for example AdDP, or releasing factor for luteinisation and follicle stimulating hormones (Fertirelin, Lecirelin). The medical preparation prepared by the method according to the invention for local administration into an open wound comprises peptide as the medical substance used to stop bleeding (Terlipressin), and Dalargin for accelerating the wound healing, optionally AdDP for local stimulation of the immune system.

**Table of peptides**

| Peptide name | Peptide sequence | Sequence no. | Possible pharmacological effect |
|---|---|---|---|
| Oxytocin | Cys-Tyr-Ile-Gln-Asn-Cys-Pro-Leu-Gly-NH2 (disulphide bridge Cys¹-Cys⁶) | SEQ ID 1 | Affects milk-ejecting activity |
| Desmopressin | Mpr-Tyr-Phe-Gln-Asn-Cys-Pro-D-Arg-Gly-NH₂ (disulphide bridge Mpr¹-Cys⁶) | SEQ ID 2 | Affects diuresis and bleeding conditions |
| Dalargin | H-Tyr-D-Ala-Gly-Phe-Leu-Arg-OH | SEQ ID 3 | Healing effects |
| Lecirelin | pGlu-His-Trp-Ser-Tyr-D-tertLeu-Leu-Arg-Pro-NHEt | SEQ ID 4 | Affects releasing factor for luteinisation and follicle stimulating hormones |
| Fertirelin | pGlu-His-Trp-Ser-Tyr-Gly-Leu-Arg-Pro-NHEt | SEQ ID 5 | Affects the releasing factor for luteinisation and follicle stimulating hormones |
| Terlipressin (Glypressin) | Gly-Gly-Gly-Cys-Tyr-Phe-Gln-Asn-Cys-Pro-Lys-Gly-NH₂ (disulphide bridge Cys¹-Cys⁶) | SEQ ID 6 | Affects blood pressure |
| Lysipressin (8-Lys-vasopressin) | Cys-Tyr-Phe-Gln-Asn-Cys-Pro-Lys-Gly-NH₂ (disulphide bridge Cys¹-Cys⁶) | SEQ ID 7 | Affects blood pressure |
| AdDP | Ala-igln-NH-Ad | | Antiviral agent and adjuvant |
| | Ac-Arg-Gly-Asp-Gly-Gly-Gly-Nle-OH | SEQ ID 8 | Affects adhesive properties *(in vitro)* |
| | Fmoc-Arg-Gly-Asp-Gly-Gly-Gly-Nle-OH | SEQ ID 9 | |
| | Ac-Gly-Gly-Gly-Arg-Gly-Asp-OH | SEQ ID 10 | |
| | Fmoc-Gly-Gly-Gly-Arg-Gly-Asp-OH | SEQ ID 11 | |
| | Ac-Arg-Gly-Asp-Ahx-Ahx-Nle-OH | SEQ ID 12 | |
| | Fmoc-Arg-Gly-Asp-Ahx-Ahx-Nle-OH | SEQ ID 13 | |
| | Ac-Ahx-Ahx-Arg-Gly-Asp-OH | SEQ ID 14 | |
| | Fmoc-Ahx-Ahx-Arg-Gly-Asp-OH | SEQ ID 15 | |

### Brief description of the drawings

The enclosed Fig. 1 shows hyaluronan where R = H+ or Na+ and n is equal to 100 to 12500.
Fig. 2 shows the structural formula of alginate.
Fig. 3 shows the structural formula of cellulose.
Fig. 4 shows the structural formula of the medical preparation prepared by the method according to the invention, where **A** is the medical substance, **S** is the way of linking the medical substance with a carrier, N is the carrier based on hyaluronic acid and/or palmitoyl hyaluronan and/or formyl hyaluronan, m is 10 to 1250, n is 100 to 12500.
Fig. 5 shows the structural formula of hyaluronic acid and/or palmitoyl hyaluronan and/or formyl hyaluronan with the bound peptide, where HYA is hyaluronic acid and/or palmitoyl hyaluronan and/or formyl hyaluronan and the peptide comprises 7 lysine units arranged in a dendrimer structure.
Fig. 6 shows Dil stained NHDF cells adhered to a fibre of native HYA comprising RGD motives. The images were obtained by means of fluorescent microscope after 48 hours of cultivation.
Fig. 7 shows changes in the presence of non-adhered Dil stained NHDF cells population. The images were obtained by means of fluorescent microscope after 1, 2, 4, and 7 days of cultivation.

The conjugate of peptide with the material based on hyaluronan according to the invention was successfully prepared and tested by the authors in the applicant's facilities - Contipro a.s., Dolní Dobruč, CZ.

### Preferred embodiments of the invention

The fibre from the native HYA and nonwoven fabric from the native HYA were prepared according to the process described in the patent WO/2013/167098. The fibre from palmitoyl HYA was prepared according to the process described in the patent WO/2014/082611.

In the following examples, the term "equivalent (eq.)" relates to a repetitive unit of the respective form of hyaluronan. The percentage is per volume, if not stated otherwise.

The molecular weight of hyaluronan starting forms is the weight average molecular weight determined by the SEC-MALLS method.

In the examples, for the purposes of subsequent analysis only, norleucin, as the non-essential amino acid, was bound first to easily determine proportional occurrence of amino acids. However, norleucin bonding is not the necessary condition and is not intended to limit the scope of the invention.

### Example 1

### Anchoring of Fmoc-Nle-OH to the fibre of native HYA

1 m of the fibre of native HYA (12 mg, 0.03 mmol, Mw = 3.10⁵ g.mol⁻¹) was placed into a 2ml syringe reactor equipped with a frit; the fibre was repeatedly washed with DMF. The solution of 3 eq. of Fmoc-Nle-OH, 3 eq. of OxymaPure and 0.3 eq. of DMAP in 0.5 ml anhydrous DMF was prepared outside the reactor; after dissolving all of the components 3 eq. of DIC were added to activate the carboxyl group of an amino acid. This solution was transferred into the reactor to the fibre. The reaction proceeded at the temperature of 18 to 23 °C for the next 20 hours and it was terminated by filtering the reaction solution off. The fibre was then washed with 3 x 1.5 ml DMF, 3 x 1.5 ml DCM, 3 x 1.5 ml IPA, 3 x 1.5 ml DCM, 3 x 1.5 ml DEE.

The yield of the condensation reaction was determined by Fmoc-release test as the substitution S = 0.01862 mmol/g.

### Example 2

### Preparation of H-Nle-O-HYA

1 m of the fibre of Fmoc-Nle-O-HYA prepared according to the Example 1 was placed into a 2ml syringe reactor equipped with a frit; the fibre was repeatedly washed with DMF. Fmoc protecting group was cleaved off by means of adding 1.5 ml of 20% solution of piperidine in DMF into the reactor to the fibre. The reaction proceeded for 5 min at the temperature of 18 to 23°C. The reaction was repeated while extending the time of cleavage to 20 min and then it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DEE.

The reaction is quantitative. Releasing of the amino group was confirmed by performing the ninhydrin confirmation reaction (Kaiser test).

### Example 3

### Anchoring of Fmoc-Nle-OH to the fibre of native HYA

1 m of fibre of native HYA (12 mg, 0.03 mmol, Mw = 3.10⁵ g.mol⁻¹) was placed into a 2ml syringe reactor equipped with a frit, the fibre was repeatedly washed with THF. A solution of 3 eq. of Fmoc-Nle-OH, 3 eq. of OxymaPure and 0.3 eq. of DMAP in 0.5 ml THF; after dissolving all the components 3 eq. of DIC were added to activate the carboxyl group of the amino acid. This solution was transferred into the reactor with the fibre. The reaction proceeded at the temperature of 18 to 23°C for the next 20 hours and it was terminated by filtering the reaction solution off. The fibre was then washed with 3 x 1.5 ml of THF, 3 x 1.5 ml of DCM, 3 x 1.5 ml of IPA, 3 x 1.5 ml of DCM, 3 x 1.5 ml of DEE. The yield of the condensation reaction was determined by Fmoc-release test as the substitution S = 0,01759 mmol/g.

### Example 4

### Anchoring of Fmoc-Nle-OH to the fibre of native HYA

1 m of the fibre of native HYA (12 mg, 0.03 mmol, Mw = 3.10⁵ g.mol⁻¹) was placed into a 2ml syringe reactor equipped with a frit; the fibre was repeatedly washed with DMF. A solution of 1 eq. of Fmoc-Nle-OH, 1 eq. of OxymaPure, and 0.3 eq. of DMAP in 0.5 ml of anhydrous DMF was prepared outside the reactor; after dissolving all of the components 1 eq. of DIC was added to activate the carboxyl group of the amino acid. This solution was transferred into the reactor to the fibre. The reaction proceeded at the temperature of 18 to 23°C for the next 20 hours and it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DEE.

The yield of the condensation reaction was determined by Fmoc-release test as the substitution S = 0.01804 mmol/g.

### Example 5

### Preparation of Fmoc-[Lys(BOC)]₄-Nle-O-HYA

1 m of H-Nle-O-HYA prepared according to the Example 2 was placed into a 2ml syringe reactor equipped with a frit; the fibre was repeatedly washed with DMF. The solution of 3 eq. of Fmoc-Lys(Boc)-OH, and 3 eq. of OxymaPure in 0.5 ml of anhydrous DMF was prepared outside the reactor; after dissolving all of the components 3 eq. of DIC were added to activate the carboxyl group of the amino acid. This solution was transferred into the reactor to the fibre. The reaction proceeded at the temperature of 18 to 23°C for the next 20 hours and it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. The Fmoc protecting group was cleaved off by means of adding 1.5 ml of 20% solution of piperidine in DMF into the reactor with the fibre. The reaction proceeded for 5 min at the temperature of 18 to 23°C. The reaction was repeated while extending the cleavage time to 20 min and then it was terminated by filtering the reaction solution off. Then the fibre was washed with 3 x 1.5 ml of DMF, 3 x 1.5 ml of DCM, 3 x 1.5 ml of IPA, 3 x 1.5 ml of DCM, 3 x 1.5 ml of DMF. This procedure was repeated 4 times.

The yield of the condensation reaction was determined by Fmoc-release test as the substitution in mmol/g. The product composition (**n_{found}**) was confirmed by amino acids analysis as presented in Table 2. It is a serial synthesis performed by a step-by-step method; however, this procedure was repeated for all said derivatives. The individual rows show the composition of the prepared conjugate after finishing said cycle; i.e. after finishing the procedure described in Example 3 the product having the composition described in the first row is obtained. Then the procedure is repeated, and the product described in the second row is obtained, etc.

**Table 2**

| **Compound** | **S [mmol/g]** | **n_{calculated} [nmol]** | **n_{found} [nmol]** |
|---|---|---|---|
| H-Lys(Boc)-Nle-O-HYA | 0.01839 | 0.290 | 0.295 |
| H-Lys(Boc)-Lys(Boc)-Nle-O-HYA | 0.01863 | 0.576 | 0.584 |
| H-Lys(Boc)-Lys(Boc)-Lys(Boc)-Nle-O-HYA | 0.01888 | 0.870 | 0.876 |
| H-Lys(Boc)-Lys(Boc)-Lys(Boc)-Lys(Boc)-Nle-O-HYA | 0.01856 | 1.189 | 1.194 |

### Example 6

### Preparation of a peptide comprising 7 lysine units arranged in a dendrimer structure - use of a native HYA fibre

1 m of H-Nle-O-HYA prepared according to the Example 2 was placed into a 2ml syringe reactor equipped with a frit; the fibre was repeatedly washed with DMF. The solution of 3 eq. of Fmoc-Lys(Fmoc)-OH, and 3 eq. of OxymaPure in 0.5 ml anhydrous DMF was prepared outside the reactor; after dissolving all the components 3 eq. of DIC were added to activate the carboxyl group of the amino acid. This solution was transferred into the reactor with the fibre. The reaction proceeded at the temperature of 18 to 23°C for the next 20 hours. The reaction was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. The Fmoc protecting group was cleaved off by means of adding 1.5 ml of 20% solution of piperidine in DMF into the reactor with the fibre. The reaction proceeded for 5 min at the temperature of 18 to 23°C. The reaction was repeated while extending the cleavage time to 20 min. The reaction was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. This procedure was repeated 2 times.

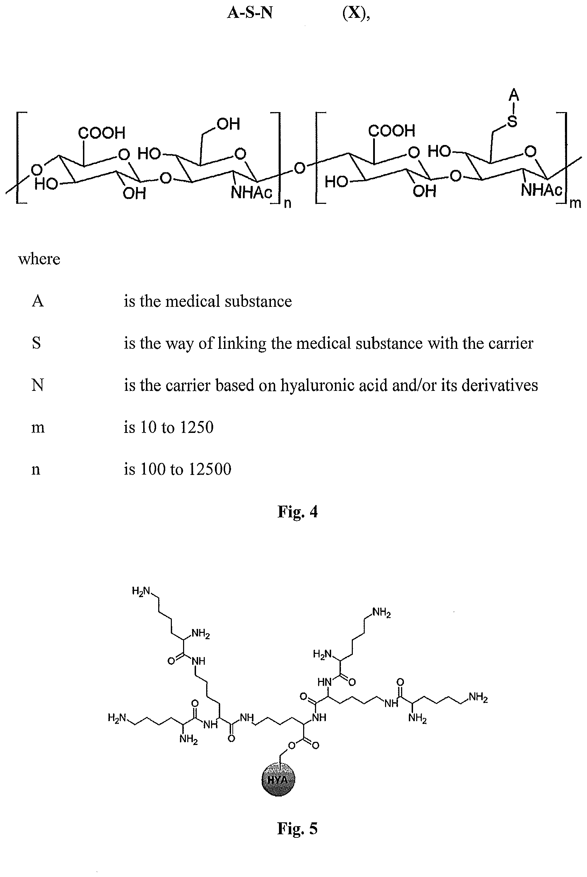

The reaction was monitored by the Fmoc-release test. The yield was determined by the Fmoc-release test. The composition of the product, shown in Fig. 5, was confirmed by an amino acid analysis, as presented in Table 3. It is a serial synthesis performed by step-by-step method; however, this procedure was repeated for all said derivatives. The individual rows show the composition of the prepared conjugate after finishing said cycle; i.e. after finishing the procedure described in Example 3 the product having the composition described in the first row is obtained. Then the procedure is repeated, and the product described in the second row is obtained, etc.

**Table 3**

| **Compound** | **S [mmol/g]** | **n_{calculated} [nmol]** | **n_{found} [nmol]** |
|---|---|---|---|
| H-Lys-Nle-O-HYA | 0.03381 | 0.25 | 0.262 |
| H-Lys₃-Nle-O-HYA | 0.07169 | 0.77 | 0.799 |
| H-Lys₇-Nle-O-HYA | 0.14211 | 1.84 | 1.876 |

### Example 7

### Preparation of a peptide comprising 7 alanine units on the fibre of native HYA

1 m of H-Nle-O-HYA prepared according to the Example 2 was placed into a 2ml syringe reactor equipped with a frit; the fibre was repeatedly washed with DMF. A solution of 3 eq. of Fmoc-Lys(Boc)-OH, and 3 eq. of OxymaPure in 0.5 ml of anhydrous DMF was prepared outside the reactor; after dissolving all of the components 3 eq. of DIC were added to activate the carboxyl group of the amino acid. This solution was transferred into the reactor to the fibre. The reaction proceeded at the temperature of 18 to 23 °C for the next 20 hours. The reaction was terminated by filtering the reaction solution off. Then the fibre was washed with 3 x 1.5 ml of DMF, 3 x 1.5 ml of DCM, 3 x 1.5 ml of IPA, 3 x 1.5 ml of DCM, 3 x 1.5 ml of DMF. Cleavage of Fmoc protecting group was performed by adding 1.5 ml of 20% solution of piperidine in DMF into the reactor to the fibre. The reaction proceeded for 5 min at the temperature of 18 to 23 °C. The reaction was repeated while extending the cleavage time to 20 min and it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. This procedure was repeated 7 times for Fmoc-Ala-OH.

The yield of the condensation reaction was determined by Fmoc-release test as the substitution in mmol/g. The product composition was confirmed by an amino acids analysis as the proportional occurrence of individual amino acids. The purity of the material was determined by means of MS-HPLC after degradation of the material. The results of the individual analysis are shown in the following Table 4:

**Table 4**

| **Compound** | **S [mmol/g]** | **Amino acid** | | | **Purity [%]** |
|---|---|---|---|---|---|
| | | **Ala** | **Lys** | **Nle** | |
| H-(Ala)₇-Lys(Boc)-Nle-O-HYA | 0.01798 | 7.28 | 1.03 | 1 | 86 |

### Example 8

### Anchoring of Fmoc-Nle-OH to the fibre of palmitoyl HYA

1 m of the fibre from palmitoyl HYA (15 mg, 0.03 mmol, Mw = 3.10⁵ g.mol⁻¹) was placed into a 2ml syringe reactor equipped with a frit; the fibre was repeatedly washed with DMF. A solution of 3 eq. of Fmoc-Nle-OH, and 3 eq. of OxymaPure and 0.3 eq. of DMAP in 0.5 ml anhydrous DMF was prepared outside the reactor; after dissolving all of the components, 3 eq. of DIC were added to activate the carboxyl group of the amino acid. This solution was transferred into the reactor to the fibre. The reaction proceeded at the temperature of 18 to 23°C for the next 20 hours and it was terminated by filtering the reaction solution off. Then the fibre was washed with 3 x 1.5 ml of DMF, 3 x 1.5 ml of DCM, 3 x 1.5 ml of IPA, 3 x 1.5 ml of DCM, 3 x 1.5 ml of DMF.

The yield of the condensation reaction was determined by Fmoc-release test as the substitution S = 0.01029 mmol/g.

### Example 9

### Preparation of H-Nle-O-palmitoyl HYA

1 m of Fmoc-Nle-O-*palmitoyl*HYA prepared according to Example 8 was placed into a 2ml syringe reactor equipped with a frit; the fibre was repeatedly washed with DMF. Fmoc protecting group was cleaved off by means of adding 1.5 ml of 20% solution of piperidine in DMF into the reactor to the fibre. The reaction proceeded at the temperature of 18 to 23 °C for 5 min. The reaction was repeated while extending the time of cleavage to 20 min and then it was terminated by filtering the reaction solution off. Then the fibre was washed with 3 x 1.5 ml of DMF, 3 x 1.5 ml of DCM, 3 x 1.5 ml of IPA, 3 x 1.5 ml of DCM, 3 x 1.5 ml of DEE.

The reaction is quantitative. Releasing of the amino group was confirmed by performing the ninhydrin confirmation reaction (Kaiser test).

### Example 10

### Preparation of Fmoc-[Lys(Boc)]₄-Nle-O-palmitoylHYA

1 m of H-Nle-O-*palmitoyl*HYA prepared according to the Example 9 was placed into a 2ml syringe reactor equipped with a frit; the fibre was repeatedly washed with DMF. A solution of 3 eq. of Fmoc-Lys(Boc)-OH, and 3 eq. of OxymaPure in 0.5 ml anhydrous DMF was prepared outside the reactor; after dissolving all of the components 3 eq. of DIC were added to activate the carboxyl group of the amino acid. This solution was transferred into the reactor to the fibre. The reaction proceeded at the temperature of 18 to 23 °C for the next 20 hours and it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. Cleavage of Fmoc protecting group was performed by adding 1.5 ml of 20% solution of piperidine in DMF into the reactor to the fibre. The reaction proceeded for 5 min at the temperature of 18 to 23°C. The reaction was repeated while extending the cleavage time to 20 min and then it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. This procedure was repeated 4 times.

The yield of the condensation reaction was determined by Fmoc-release test as the substitution in mmol/g. The composition of the product (**n_{found}**) was confirmed by amino acid analysis, as presented in Table 5 below. It is a serial synthesis performed by the step-by-step method; however, this procedure was repeated for all the derivatives. The individual rows show the composition of the prepared conjugate after finishing the said cycle; i.e. after finishing the procedure described in the Example 3 the product having the composition described in the first row is obtained. Then the procedure is repeated, and the product described in the second row is obtained, etc.

**Table 5**

| **Compound** | **S [mmol/g]** | **N_{calculate d} [nmol]** | **N_{foundo} [nmol]** |
|---|---|---|---|
| H-Lys(Boc)-Nle-O-*palmitoyl*HYA | 0.01044 | 0.157 | 0.152 |
| H-Lys(Boc)-Lys(Boc)-Nle-O-*palmitoyl*HYA | 0.01057 | 0.314 | 0.318 |
| H-Lys(Boc)-Lys(Boc)-Lys(Boc)-Nle-O-*palmitoyl*HYA | 0.01061 | 0.478 | 0.469 |
| H-Lys(Boc)-Lys(Boc)-Lys(Boc)-Lys(Boc)-Nle-O-*palmitoyl*HYA | 0.01053 | 0.631 | 0.619 |

### Example 11

### Preparation of a peptide comprising 7 lysine units arranged in a dendrimer structure - use of the palmitoyl HYA fibre

1 m of H-Nle-O-*palmitoyl*HYA prepared according to the Example 9 was placed into the 2ml syringe reactor equipped with a frit; the fibre was repeatedly washed with DMF. A solution of 3 eq. of Fmoc-Lys(Boc)-OH, and 3 eq. of OxymaPure in 0.5 ml anhydrous DMF was prepared outside the reactor; after dissolving all components, 3 eq. of DIC were added to activate the carboxyl group of the amino acid. This solution was transferred into the reactor to the fibre. The reaction proceeded at the temperature of 18 to 23 °C for the next 20 hours. The reaction was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. Cleavage of Fmoc protecting group was performed by adding 1.5 ml of 20% solution of piperidine in DMF into the reactor to the fibre. The reaction proceeded for 5 min at the temperature of 18 to 23 °C. The reaction was repeated while extending the cleavage time to 20 min. The reaction was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DMF. This procedure was repeated 2 times.

The reaction was monitored by Fmoc-release test. The yield was determined by Fmoc-release test. The composition of the product, shown in Fig. 5, where HYA denotes palmitoyl hyaluronan, was confirmed by amino acid analysis, as presented in Table 6. It is a serial synthesis performed by the step-by-step method; however, this procedure was repeated for all the said derivatives. The individual rows show the composition of the prepared conjugate after finishing said cycle; i.e. after finishing the procedure described in the Example 3 the product having the composition described in the first row is obtained. Then the procedure is repeated, and the product described in the second row is obtained, etc.

**Table 6**

| **Compound** | **S [mmol/g]** | **N_{calculated} [nmol]** | **Nfound [nmol]** |
|---|---|---|---|
| H-Lys-Nle-O-*palmitoyl*HYA | 0.00984 | 0.16 | 0.154 |
| H-Lys₃-Nle-O-*palmitoyl*HYA | 0.02130 | 0.49 | 0.501 |
| H-Lys₇-Nle-O-*palmitoyl*HYA | 0.04265 | 1.12 | 1.113 |

### Example 12

### Anchoring Fmoc-Lys-NH₂ to formylHYA fibre

1 m of *formyl*HYA fibre (11 mg, 0.028 mmol, Mw = 3.10⁵ g.mol⁻¹) was placed into a 2ml syringe reactor equipped with a frit; the fibre was repeatedly washed with DMF. The solution of 3 eq. of Fmoc-Lys-NH₂ in 0.5 ml of anhydrous DMF was prepared outside the reactor and transferred into the reactor to the fibre. The reaction proceeded at the temperature of 18 to 23°C for the next 20 hours. Then 3 eq. of picBH3 were added and the reaction proceeded at the temperature of 18 to 23 °C for the next 24 hours, then it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DEE. Cleavage of Fmoc protecting group was performed by adding 1.5 ml of 20% solution of piperidine in DMF into the reactor to the solution. The reaction proceeded for 5 min at the temperature of 18 to 23 °C. The reaction was repeated while extending the cleavage time to 20 min and then it was terminated by filtering the reaction solution off. The fibre was then washed with 3 x 1.5 ml of DMF, 3 x 1.5 ml of DCM, 3 x 1.5 ml of IPA, 3 x 1.5 ml of DCM, 3 x 1.5 ml of DEE.

Releasing of the amino group was confirmed by performing a ninhydrin confirmation reaction (Kaiser test).

### Example 13

### Anchoring the peptide with RGD motif to the fibre of native HYA

1 m of native HYA fibre (12 mg, 0.03 mmol, Mw = 3.10⁵ g.mol⁻¹) was placed into a 2ml syringe reactor equipped with a frit; the fibre was repeatedly washed with DMF. A solution of 3 eq. of a peptide having the appropriate sequence, 3 eq. of OxymaPure in 0.3 eq. of DMAP in 0.5 ml of anhydrous DMF was prepared outside the reactor; after dissolving all the components 3 eq. of DIC were added to activate the carboxyl group of the amino acid. This solution was transferred into the reactor to the fibre. The reaction proceeded at the temperature of 18 to 23 °C for the next 20 hours. The reaction was terminated by filtering the reaction solution off. Then the fibre was washed with 3 x 1.5 ml of DMF, 3 x 1.5 ml of DCM, 3 x 1.5 ml of IPA, 3 x 1.5 ml of DCM, 3 x 1.5 ml of DEE.

The yield of the condensation reaction was determined by Fmoc-release test as the substitution in mmol/g. The composition of the product was confirmed by amino acid analysis as the proportion of individual amino acids. The purity of the material was determined by means of MS-HPLC after the degradation of the material. The results of the individual analyses are shown in the following Table 7:

**Table 7**

| **Compound** | **S [mmol/g]** | **Amino acid** | | | | | **Purity [%]** |
|---|---|---|---|---|---|---|---|
| | | **Ahx** | **Asp** | **Gly** | **Nle** | **Arg** | |
| Ac-Arg-Gly-Asp-Gly-Gly-Gly-Nle-O-HYA | - | - | 1.04 | 3.89 | 1 | 1.03 | 90 |
| H-Arg-Gly-Asp-Gly-Gly-Gly-Nle-O-HYA | 0.01802 | - | 1.01 | 3.91 | 1 | 0.97 | 82 |
| H-Gly-Gly-Gly-Arg-Gly-Asp-Nle-O-HYA | 0.01832 | - | 1.04 | 4.07 | 1 | 1.08 | 85 |
| Ac-Arg-Gly-Asp-Ahx-Ahx-Nle-O-HYA | - | 2.20 | 1.04 | 1.06 | 1 | 1.01 | 95 |
| H-Arg-Gly-Asp-Ahx-Ahx-Nle-O-HYA | 0.01843 | 2.27 | 0.93 | 1.05 | 1 | 1.00 | 90 |
| Ac-Ahx-Ahx-Arg-Gly-Asp-Nle-O-HYA | - | 2.21 | 1.03 | 1.26 | 1 | 1.01 | 86 |
| H-Ahx-Ahx-Arg-Gly-Asp-Nle-O-HYA | 0.01831 | 2.31 | 0.98 | 1.05 | 1 | 1.02 | 89 |

### Example 14

### Anchoring the peptide having RGD motif to the native HYA nonwoven fabric

5 squares of the side of 5 mm made of nonwoven fabric of native HYA (10.5 mg, 0.03 mmol, Mw = 1.04x10⁶ g.mol⁻¹) were placed into a 2ml syringe reactor equipped with a frit; the material was repeatedly washed with DMF. A solution of 3 eq. of a peptide having the appropriate sequence and 3 eq. of OxymaPure in 0.5 ml of anhydrous DMF was prepared outside the reactor; after dissolving all of the components, 3 eq. of DIC were added to activate the carboxyl group of the amino acid. This solution was transferred into the reactor to the material. The reaction proceeded at the temperature of 18 to 23 °C for the next 20 hours. The reaction was terminated by filtering the reaction solution off. Then the fabric was washed with 3 x 1.5 ml of DMF, 3 x 1.5 ml of DCM, 3 x 1.5 ml of IPA, 3 x 1.5 ml of DCM, 3 x 1.5 ml of DEE.

The yield of the condensation reaction was determined by Fmoc-release test as the substitution in mmol/g. The composition of the product was confirmed by an amino acid analysis as the proportional occurence of the individual amino acids. The purity of the material was determined by means of MS-HPLC after the degradation of the material. The results of the individual analysis are presented in the following Table 8:

**Table 8**

| **Compound** | **S [mmol/g]** | **Amino acid** | | | | | **Purity [%]** |
|---|---|---|---|---|---|---|---|
| | | **Ahx** | **Asp** | **Gly** | **Nle** | **Arg** | |
| H-Arg-Gly-Asp-Gly-Gly-Gly-Nle-O-HYA | 0.01802 | - | 1.04 | 3.94 | 1 | 1.01 | 85 |
| H-Gly-Gly-Gly-Arg-Gly-Asp-Nle-O-HYA | 0.01832 | - | 1.06 | 4.14 | 1 | 1.07 | 76 |
| Ac-Arg-Gly-Asp-Ahx-Ahx-Nle-O-HYA | - | 2.17 | 0.96 | 1.05 | 1 | 1.04 | 95 |
| H-Arg-Gly-Asp-Ahx-Ahx-Nle-O-HYA | 0.01843 | 2.15 | 1.01 | 1.12 | 1 | 1.06 | 88 |

### Example 15

### Anchoring the peptide Fmoc-(VPGLG)₂-OH to a fibre of native HYA

1 m of native HYA fibre (12 mg, 0.03 mmol, Mw = 3.10⁵ g.mol⁻¹) was placed into a 2ml syringe reactor equipped with a frit; the fibre was repeatedly washed with DMF. A solution of 3 eq. of the peptide Fmoc-(VPGLG)₂-OH, 3 eq. of OxymaPure, and 0.3 eq. of DMAP in 0.5 ml of anhydrous DMF was prepared outside the reactor; after dissolving all of the components, 3 eq. of DIC were added to activate the carboxyl group of the amino acid. This solution was transferred into the reactor to the fibre. The reaction proceeded at the temperature of 18 to 23 °C for further 20 hours, and it was terminated by filtering the reaction solution off. Then the fibre was washed with 3 x 1.5 ml of DMF, 3 x 1.5 ml of DCM, 3 x 1.5 ml of IPA, 3 x 1.5 ml of DCM, 3 x 1.5 ml of DEE.

The yield of the condensation reaction was determined by Fmoc-release test as the substitution in mmol/g. The composition of the product was confirmed by an amino acid analysis as the proportional occurrence of the individual amino acids. The purity of the material was determined by means of MS-HPLC after the degradation of the material. The results of the individual analysis are shown in the following Table 9:

**Table 9**

| **Compound** | **S [mmol/g]** | **Amino acid** | | | | **Purity [%]** |
|---|---|---|---|---|---|---|
| | | **Gly** | **Leu** | **Pro** | **Val** | |
| H-(Val-Pro-Gly-Leu-Gly)₂-O-HYA | 0.01809 | 2.37 | 1.26 | 1.13 | 1 | 85 |

### Example 16

### Anchoring Dalagrin to the fibre of native HYA

1 m of native HYA fibre (12 mg, 0.03 mmol, Mw = 3.10⁵ g.mol⁻¹) was placed into a 2ml syringe reactor equipped with a frit; the fibre was repeatedly washed with DMF. A solution of 3 eq. of the peptide Fmoc-Dalagrin, 3 eq. of OxymaPure, and 0.3 eq. of DMAP in 0.5 ml of anhydrous DMF was prepared outside the reactor; after dissolving all of the components, 3 eq. of DIC were added to activate the carboxyl group of the amino acid. This solution was transferred into the reactor to the fibre. The reaction proceeded at the temperature of 18 to 23°C for further 20 hours, and it was terminated by filtering the reaction solution off. Then the fibre was washed with 3 x 1.5 ml of DMF, 3 x 1.5 ml of DCM, 3 x 1.5 ml of IPA, 3 x 1.5 ml of DCM, 3 x 1.5 ml of DEE.

The yield of the condensation reaction was determined by Fmoc-release test as the substitution in mmol/g. The composition of the product was confirmed by an amino acid analysis as the proportional occurrence of the individual amino acids. The purity of the material was determined by means of MS-HPLC after the degradation of the material. The results of the individual analysis are shown in the following Table 10:

**Table 10**

| **Compound** | **S [mmol/g]** | **Amino acid** | | | | | | | **Purity [%]** |
|---|---|---|---|---|---|---|---|---|---|
| | | **ala** | **Arg** | **Gly** | **Nle** | **Leu** | **Phe** | **Tyr** | |
| H-Tyr-ala-Gly-Phe-Leu-Arg-Nle-O-HYA | 0.01894 | 0.99 | 0.99 | 0.96 | 1 | 0.98 | 0.96 | 0.99 | 93 |

### Example 17

### In vitro cell adhesion on the fibre comprising RGD motif

The fibre of native HYA comprising RGD motif, prepared according to the procedure described in Example 14, and control fibres were cut into 1 cm pieces and transferred onto a non-adhesive panel with 24 wells. Primary human fibroblasts (NHDF) were pre-marked with the fluorescent dye DiI (Exₘₐₓ/Emₘₐₓ 549/565) and inoculated, in the amount of 10⁵ cells, into the wells filled with the tested samples. Then the panel was shaken by a shaker (5 hrs/ 160 rpm) to keep the cells in suspension, under cultivation conditions (37 °C, 5% CO₂). After 24 h of cultivation the fibres were transferred into a well with fresh cultivation medium. Then the cells were observed and detected by fluorescent microscope Nikon Ti-Eclipse with the use of TRITC filter (Exₘₐₓ/Emₘₐₓ, 549/565). The fibroblasts proliferation was monitored for 7 days of cultivation.

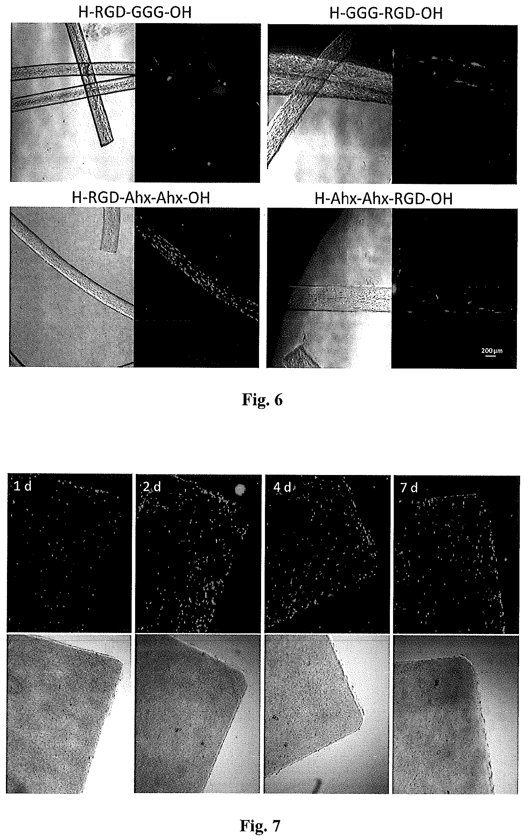

Human dermal fibroblasts adhered and proliferated during the time only on the fibre with the peptide H-Arg-Gly-Asp-Ahx-Ahx-Nle-OH bonded to HYA via 2 units od 6-aminohexanoic acid that form a firm and elastic linker and thus make the RGD peptide accessible for cell adhesion receptors. On the contrary, the peptide H-Ahx-Ahx-Arg-Gly-Asp-Nle-OH, as well as the linking of peptides H-Arg-Gly-Asp-Gly-Gly-Gly-Nle-OH and H-Gly-Gly-Gly-Arg-Gly-Asp-Nle-OH via triglycine linker, did not support the cell adhesion, as can be seen in Fig. 6. Based on these data we can assume that it is the RGD motif which supports the cell adhesion, being the minimum domain that can be recognized by cell receptors, eventually the Ahx-linker. The positive effect of Ahx-linker was then displaced by testing the fibres with the bonded peptide comprising RGD or RGD motif, where NHDF adhered exclusively on the peptide with RGD.

### Example 18

### In vitro cell adhesion on a nonwoven fabric of native HYA comprising RGD motif

Nonwoven fabric of HYA with an anchored peptide H-Arg-Gly-Asp-Ahx-Ahx-Nle-OH prepared according to the procedure described in Example 14, and a control fabric were cut into squares of the side of 0.5 cm, and were transferred onto 24 wells plate of non-adhesive panel. Primary human fibroblasts (NHDF) were pre-marked with the fluorescent dye DiI (Exₘₐₓ/Emₘₐₓ 549/565) and inoculated, in the amount of 10⁵ cells, into the wells filled with the tested samples. Then the panel was shaken by a shaker (5 hrs/ 160 rpm) to keep the cells in suspension, under cultivation conditions (37 °C, 5% CO₂). After 24 h of cultivation the fabric was transferred into a well with a fresh culture media. Then the cells were observed and detected by the fluorescent microscope Nikon Ti-Eclipse with the use of TRITC filter (Exₘₐₓ/Emₘₐₓ, 549/565). The fibroblasts proliferation was monitored for 7 days of cultivation, as can be seen in Fig. 7.

NHDF adhered uniformly onto the fabric surface and after 48 hours they elongated in a characteristic manner.

### Industrial Applicability

The medical preparation with a carrier based on hyaluronan and/or palmitoyl hyaluronan and/or formyl hyaluronan can be used as a suitable drug form for treatment with the attached medical substance. This dosage form brings an improved prolonged activity of the medical substance bound on the solid carrier of the complex system. Suitable forms of hyaluronan with the bound medical substances can be preferably used in human and veterinary medicine.

### List of Abbreviations

- AdDP: 1-adamantylamide-(L)-alanyl-(D)-isoglutamine
- Ahx: aminohexanoic acid
- Boc: *tert*-butyloxycarbonyl
- Castro reagent: benzotriazol-1-yl-oxy-tris(dimethylamino)phosphonium hexafluoro phosphate
- DBU: 1,8-diazabicyclo[5.4.0]undec-7-ene
- DCC: *N,N'*-dicyclohexylcarbodiimide
- DCM: dichloromethane
- DEE: diethylether
- DIC: *N,N'*-diisopropylcarbodiimide
- Dil: 1,1'-dioctadecyl-3,3,3',3'-tetramethyl indocarbocyanine perchlorate
- DIPEA: *N*,*N'*-diisopropyl ethylamine
- DMAP: *N,N*-dimethyl aminopyridine
- DMF: *N,N*-dimethyl formamide
- EDC: 1-ethyl-3-(3-dimethyl aminopropyl) carbodiimide
- Fmoc: fluorenyl methyloxy carbonyl
- HATU: 1-[bis(dimethylamino) methylen]-1*H*-1,2,3-triazolo[4,5-b] pyridinium 3-oxide hexafluoro phosphate
- HBTU: 2-(1*H*-benzotriazole-1-yl)-1,1,3,3-tetramethyl uronium hexafluoro phosphate
- HOAt: 1-hydroxy-7-azabenzo triazole
- HOBt: *N*-hydroxy benztriazole
- HOSu: *N*-hydroxy succinimide
- IPA: isopropyl alcohol
- OxymaPure: ethyl (hydroxyimino) cyanoacetate
- picBH₃: picolin borane complex
- T3P: propyl phosphonic acid anhydride
- TBTU: *O*-(benzotriazole-1-yl)-*N,N,N',N'*-tetramethyl uronium tetrafluoro borate
- WSCD: 4-triethylamino dicyclohexyl carbodiimide *p*-toluen sulphonate

### Literature

1. Schanté C. E. et al.: Carbohyd Polym 85, 469 (2011).
2. Danishefsky I., Siskovic E.: Carbohyd Res 16 (1), 199 (1971).
3. Bulpitt P., Aeschlimann D.: J Biomed Mater Res 47 (2), 152 (1999).
4. Foullain N., Montanari S., Jeacomine I., Gambarelli S., Vignon M.: Carbohyd Polym 74 (3), 333 (2008).
5. Oh E., Park K., Kim K., Kim J., Yang J., Kong J.: J Control Release 141 (1), 2 (2010).
6. Magnani A., Rappuoli R., Lamponi S., Barbucci R.: Polym Advan Technol 11 (8-12), 488 (2000).
7. Bergman K., Elvingson C., Hilborn J., Svensk G., Bowden T.: Biomacromolecules 8 (7), 2190 (2007).
8. Bellini D., Topai A.: WO/2000/001733 (2000).
9. Della Valle F., Romeo A.: US4851521 (1986).
10. Pelletier S., Hubert P., Lapicque F., Payan E., Dellacherie E.: Carbohyd Polym 43 (4), 343 (2000).
11. Jeanloz R., Forchielli E.: J Biol Chem 186 (2), 495 (1950).
12. Hirano K., Sakai S., Isshikawa T., Avci F., Linhardt R., Toida T.: Carbohyd Res 340 (14), 2297 (2005).
13. Leach J., Bivens K., Patrick Jr. C., Schmidt C.: Biotechnol Bioeng 82 (5), 578 (2003).
14. Bencherif S., Srinivasan A., Horkay F., Hollinger J., Matyjaszewski K., Washburn N.: Biomaterials 29 (12), 1973 (2008).
15. Weng L., Gouldstone A., Wu Y., Chen W.: Biomaterials 29 (14), 2153 (2008).
16. Prata J., Barth T., Bencherif S., Washburn N.: Biomacromolecules 11(3), 769 (2010).
17. Laurent T., Hellsing K., Gelotte B.: Acta Chem Scand 18 (1), 274 (1964).
18. Yui N., Okano T., Sakurai Y.: J Control Release 22 (2), 105 (1992).
19. Tomihata K., Ikada Y.: Biomaterials 18 (3), 189 (1997).
20. Zhao X.: WO/2000/046253 (2000).
21. Balazs E., Leshchiner A.: US4582865 (1986).
22. Ramamurthi A., Vesely I.: J Biomed Mater Res 60 (1), 196 (2002).
23. Eun J., Kang S., Kim B., Jiang G., Il H., Sei K.: J Biomed Mater Res-A 86 (3), 685 (2008).
24. Serban M., Yang G., Prestwich G.: Biomaterials 29 (10), 1388 (2008).
25. Tomihata K., Ikada Y.: J Polym Sci Pol Chem 35 (16), 3553 (1997).
26. Crescenzi V. et al.: Carbohyd Polym 53 (3), 311 (2003).
27. Velebný V., Hrdina R., Šuláková R., Mlčochová P., Holas T., Krčmář M.: CZ302856 (2006).
28. Šmejkalová D. et al.: WO/2014/082609 (2014).
29. Huerta-Angeles G., Bobek M., Příkopová E., Šmejkalová D., Velebný V.: Carbohyd Polym 111, 883 (2014).
30. Buffa R., Velebný V., Pospíšilová L., Příkopová E., Pravda M., Nikodým P., Palek L.: WO/2010/105582 A1 (2010).
31. Eichler J. et al.: Cotton-carrier for solid phase peptide synthesis (1991).
32. Robert Marks: WO/2012/101612 A1 (2012).
33. Palmieri G., Cassani G., Fassina G.: J Chromatogr B 664, 127 (1995).
34. Morgan S. M., Al-Shamkhani A., Callant D., Schacht E., Woodley J.F., Duncan R.: Int J Pharm 122, 121 (1995).
35. Hashimoto T., Suzuki Y., Tanihara M., Kakimaru Y., Suzuki K.: Biomaterials 25, 1407 (2004).
36. Frank R., Döring R., Tetrahedron 44, 6031 (1988).
37. Eichler J., Bienert M., Stierandova A., Lébl M.: Pept Res 4 (5), 296 (1991).
38. Eichler J. et al.: Innovation and Perspectives in Solid Phase Synthesis. Birmingham, UK: SPCC, 337 - 343 (1990).
39. Burgert L., Hrdina R.. Mašek D., Velebný V.: WO/2012/089179 A1 (2012).
40. Burgert L et al.: WO/2013/167098 A2 (2013).
41. Bět'ák J. et al.: WO/2014/082610 A1 (2014).
42. Ščudlová J. et al.: WO/2014/082611 A1 (2014).
43. Foglarová M. et al.: WO/2016/141903 (2016).
44. Foglarová M. et al.: Carbohydr Polym 144, 68 (2016).

## Claims

1. A method of preparation of a medical preparation based on hyaluronan and/or palmitoyl hyaluronan and/or formyl hyaluronan according to the general formula (X):
**A-S-N** **(X),**
which is represented by the formula: 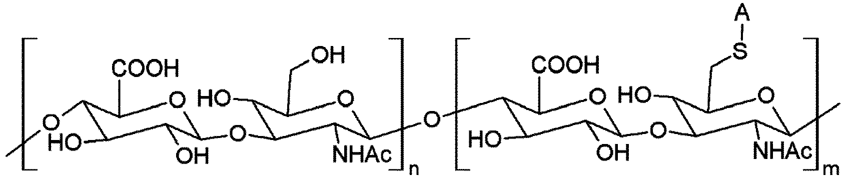 where
A is a peptide or amino acid,
S is -O-C(=O)-, -NH-, or a linear linker which is a peptide formed by hydrophobic amino acids Xaa,
m is 10 to 1250,
n is 100 to 12500,
**characterized in that**
(a) the preparation proceeds as an esterification reaction according to the Scheme 1 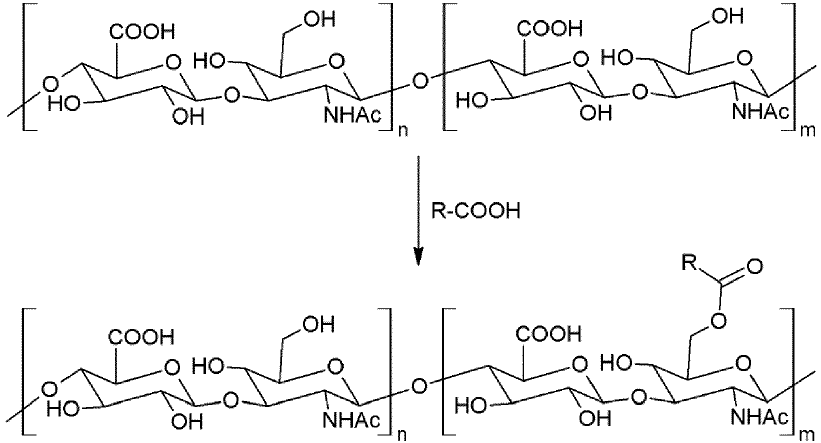 where R is a peptide or amino acid,
wherein a medical substance A comprising a terminal N-protecting group and a carboxyl group, the medical substance A being selected from the group consisting of amino acids and peptides, is first subjected to activation of the carboxyl group by a condensation agent in an aprotic polar solvent to form a reactive intermediate, that is subsequently reacted with a carrier N, the carrier N being hyaluronic acid, palmitoyl hyaluronan or formyl hyaluronan and having the molecular weight within the range of 1.5x10⁴ to 2.5x10⁶ g.mol⁻¹, and the carrier N being in the form of an endless fibre, thread, textile, thin film, staple fibre and/or nonwoven fabric, in the presence of an organic base and a catalyst, and then the terminal N-protecting group of the medical substance A bound on the carrier is cleaved off in a basic environment, to form the conjugate according to the general formula X, where S is -O-C(=O)-, or a linear linker which is a peptide formed by hydrophobic amino acids Xaa,
or
(b) the preparation proceeds as a reductive amination reaction according to the Scheme 2 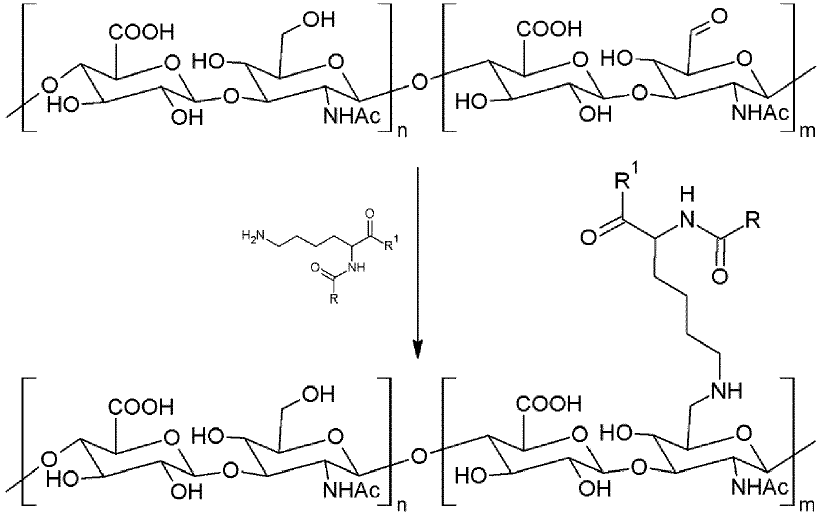 where R is an amino acid or peptide, and R¹ is an amide or methylester, wherein a medical substance A comprising a terminal N-protecting group and being selected from the group consisting of amino acids and peptides is reacted with a carrier N in a polar aprotic solvent and in the presence of a reduction agent, the carrier N being hyaluronic acid and/or palmitoyl hyaluronan and/or formyl hyaluronan and having the molecular weight within the range of 1.5x10⁴ to 2.5x10⁶ g.mol⁻¹, and the carrier N being in the form of an endless fibre, thread, textile, thin film, staple fibre and/or nonwoven fabric, and then the terminal N-protecting group of the medical substance A bound on the carrier is cleaved off in a basic environment, to form the conjugate according to the general formula X, where S is -NH-, or a linear linker which is a peptide formed by hydrophobic amino acids Xaa,
wherein for (a) and (b) the carrier N is in solid phase during the whole preparation, is insoluble in the reaction environment and is biodegradable.

2. The method according to claim 1 **characterized in that** the formation of the conjugate of hyaluronic acid and palmitoyl hyaluronan and formyl hyaluronan is performed at the temperature of 20 °C to 40 °C for 2 to 48 hours, preferably at the temperature of 22 °C to 25 °C for 24 hours.

3. The method according to any of the preceding claims **characterized in that** the aprotic polar solvent is selected from the group consisting of *N,N*-dimethyl formamide, dimethyl sulphoxide, *N*-methyl-2-pyrrolidone, acetonitrile, dichloro methane, tetrahydrofuran, 1,4-dioxane and/or the basic environment in which the N-terminal protecting group is cleaved-off is selected from the group consisting of 20% piperidine in DMF, 2% DBU in DMF, 30% tert-butylamine in DMF.

4. The method of preparation according to claim 1, **characterized in that** the medical substance A is bound to the carrier N by means of esterification, where the medical substance A is an amino acid or peptide comprising a terminal N-protecting group and a carboxyl group, wherein the medical substance A is first subjected to activation of the carboxyl group by a condensation agent in an aprotic polar solvent to form a reactive intermediate, that is subsequently reacted with the carrier N at the presence of an organic base and a catalyst, and then the terminal N-protecting group of the medical substance A bound on the carrier is cleaved off in a basic environment, to form the conjugate according to the general formula X, where S is -O-C(=O)-, or a linear linker which is a peptide formed by hydrophobic amino acids Xaa.

5. The method according to claim 4 **characterized in that** the condensation agent is selected from the group of N,N'-diisopropyl carbodiimide, N,N'-dicyclohexyl carbodiimide, 1-[bis(dimethylamino) methylen]-1H-1,2,3-triazolo[4,5-b] pyridinium 3-oxide hexafluoro phosphate, 1-ethyl-3-(3-dimethyl aminopropyl) carbodiimide, propylphosphonic acid anhydride, 2-(1H-benzotriazole-1-yl)-1,1,3,3-tetramethyl uronium hexafluoro phosphate, ethyl chloroformiate, benzotriazole-1-yl-oxy-tris(dimethylamino) phosphonium hexafluoro phosphate, O-(benzotriazole-1-yl)-N,N,N',N'-tetramethyl uronium tetrafluoroborate, 4-triethylamino-dicyclohexyl carbodiimide p-toluen sulfonate, 4-nitrophenol, N-hydroxy succinimide, 2,4,5-trichloro phenol, 2,3,4,5,6-pentafluoro phenol, 2,3,4,5,6-pentachloro phenol.

6. The method according to claim 4 **characterized in that** the organic base is selected from the group consisting of triethylamine, pyridine, morpholine, *N*-methyl morpholine, *N*,*N'*-diisopropyl ethylamine, imidazole and/or the catalyst is selected from the group consisting of ethyl(hydroxyimino) cyanoacetate, hydroxybenzo triazole, 1-hydroxy-7-azabenzotriazole, or *N,N-* dimethylamino pyridine.

7. The method according to claim 4 **characterized in that** the conjugate is further repeatedly reacted with the reactive intermediate that can be the same or different from the reactive intermediate of the previous step.

8. The method according to claim 4 **characterized in that** the condensation agent is *N*,*N'*-diisopropyl carbodiimide, the aprotic polar solvent is *N,N*-dimethyl formamide, the catalyst is the combination of ethyl (hydroxyimino) cyanoacetate and *N,N-*dimethylamino pyridine, and the basic environment is 20% piperidine in N,N-dimethyl formamide.

9. The method according to any of claims 1 to 8 **characterized in that** the amount of the medical substance A corresponds to 1 to 5 equivalents, with respect to a dimer of hyaluronic acid or of palmitoyl hyaluronan or of formyl hyaluronan.

10. The method according to any of claims 4 to 8 **characterized in that** the amount of the condensation agent corresponds to 0.1 to 5 equivalents, with respect to a dimer of hyaluronic acid or of palmitoyl hyaluronan or of formyl hyaluronan, and/or the amount of the organic base corresponds up to 10 equivalents, with respect to a dimer of hyaluronic acid or of palmitoyl hyaluronan or of formyl hyaluronan, and/or the amount of the catalyst corresponds 0.1 to 5 equivalents, with respect to a dimer of hyaluronic acid or of palmitoyl hyaluronan or of formyl hyaluronan, preferably 3 equivalents of ethyl(hydroxyimino) cyanoacetate and 0.3 equivalents of *N,N*-dimethylamino pyridine are used.

11. The method according to claim 4 **characterized in that** the amount of the medical substance A corresponds to 3 equivalents, the amount of the condensation agent corresponds to 3 equivalents, and the amount of the catalyst corresponds to 3 equivalents, with respect to a dimer hyaluronic acid or of palmitoyl hyaluronan or of formyl hyaluronan.

12. The method according to claim 4 **characterized in that** the amount of the medical substance A corresponds to 1 equivalent, the amount of the condensation agent corresponds to 1 equivalent, and the amount of the catalyst corresponds to 1 equivalent, with respect to a dimer of hyaluronic acid or of palmitoyl hyaluronan or of formyl hyaluronan.

13. The method of preparation according to any of claims 1 to 3, **characterized by that** the medical substance A bound to the carrier N by means of reductive amination in a polar aprotic solvent and in the presence of a reduction agent, and then the terminal N-protecting group of the medical substance A bound on the carrier is cleaved off in a basic environment to form the conjugate according to the general formula X, where S is -NH-, or a linear linker which is a peptide formed by hydrophobic amino acids Xaa.

14. The method according to claim 13 **characterized in that** the conjugate is further repeatedly reacted with the medical substance A that can be the same or different from the medical substance A of the previous step.

15. The method of preparation according to claim 13, **characterized in that** the polar aprotic solvent is DMF, the reduction agent is picBH₃, and the basic environment is 20% solution of piperidine in DMF.

16. The method according to claim 1 **characterized in that** the medical substance A is bound to the carrier via a linear linker Xaa-Ahx-Ahx-Xaa.

## Patentansprüche

1. Ein Verfahren zur Vorbereitung eines medizinischen Präparats auf der Basis von Hyaluronat und/oder Palmitoyl-Hyaluronat und/oder Formyl-Hyaluronat nach der allgemeinen Formel (X):
**A-S-N** **(X),**
die mittels der nachfolgenden Formel ausgedrückt ist: 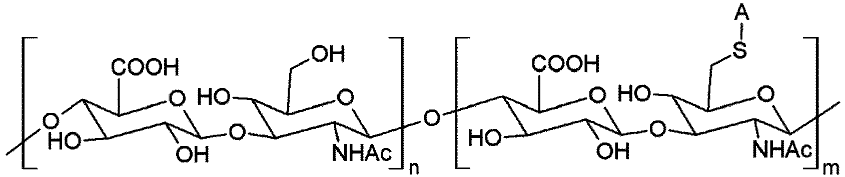 in der
A ein Peptid oder eine Aminosäure ist,
S eine -O-C(=O)- oder -NH- oder ein linearer Linker, welcher von einem Peptid dargestellt ist, das von Aminosäuren Xaa gebildet ist,
m im Bereich von 10 bis 1250 liegt,
n im Bereich von 100 bis 12500 liegt,
**dadurch gekennzeichnet, dass**
(a) die Vorbereitung als eine Veresterungsreaktion nach dem Schema 1 erfolgt 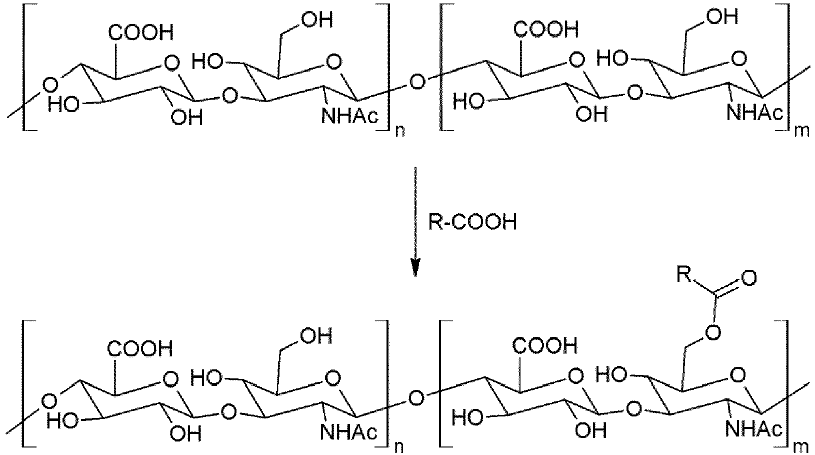 in dem R ein Peptid oder eine Aminosäure ist,
wobei zunächst eine medizinische Substanz A, die eine terminale N-schützende Gruppe sowie eine Carboxylgruppe umfasst, wobei diese medizinische Substanz A aus der von Aminosäuren und Peptiden bestehenden Gruppe ausgewählt ist, einer Aktivierung der Carboxylgruppe mittels eines Kondensierungsreagenz in einem aprotischen polaren Lösungsmittel unter Bildung eines reaktiven Zwischenprodukts unterzogen wird, welches Zwischenprodukt anschliessend mit einem N-Träger reagieren gelassen wird, wobei der N-Träger von Hyaluronsäure, Palmitoyl-Hyaluronat oder Formyl-Hyaluronat gebildet ist und ein Molekulargewicht im Bereich von 1,5x10⁴ bis 2,5x10⁶ g.mol⁻¹ aufweist und wobei der N-Träger in der Form einer endlosen Faser, eines Fadens, einer Textilie, eines dünnen Films, einer Staple-Faser und/oder einer nicht gewebten Faserbahn vorliegt, wobei die letztere Reaktion beim Vorhandensein einer organischen Base und eines Katalysators erfolgt, und im Anschluss daran die an den Träger gebundene terminale N-schützende Gruppe der medizinische Substanz A in einer basischen Umgebung gespaltet wird, um das Konjugat nach der allgemeinen Formel X zu bilden, in der S -O-C(=O)- oder ein linearer Linker ist, welcher von einem Peptid dargestellt ist, das von hydrophobischen Aminosäuren Xaa gebildet ist,
oder
(b) die Vorbereitung als eine reduzierenden Aminierungsreaktion nach dem Schema 2 erfolgt 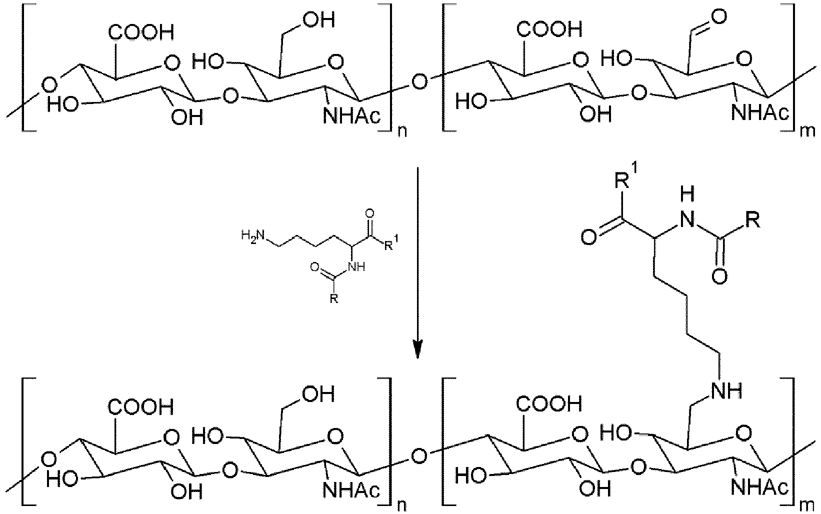 in dem R ein Peptid oder eine Aminosäure ist und R¹ ein Amid oder Methylester ist, wobei eine medizinische Substanz A, die eine terminale N-schützende Gruppe umfasst und aus der von Aminosäuren und Peptiden bestehenden Gruppe ausgewählt ist, einer Reaktion mit einem N-Träger in einem aprotischen polaren Lösungsmittel beim Vorhandensein eines reduzierenden Reagenz unterzogen wird, wobei der N-Träger von Hyaluronsäure und/oder Palmitoyl-Hyaluronat und/oder Formyl-Hyaluronat gebildet ist und ein Molekulargewicht im Bereich von 1,5x10⁴ bis 2,5x10⁶ g.mol⁻¹ aufweist und wobei der N-Träger in der Form einer endlosen Faser, eines Fadens, einer Textilie, eines dünnen Films, einer Staple-Faser und/oder einer nicht gewebten Faserbahn vorliegt, und im Anschluss daran die an den Träger gebundene terminale N-schützende Gruppe der medizinische Substanz A in einer basischen Umgebung gespaltet wird, um ein Konjugat nach der allgemeinen Formel X zu bilden, in der S -NH- oder ein linearer Linker ist, welcher von einem Peptid dargestellt ist, das von hydrophobischen Aminosäuren Xaa gebildet ist,
wobei sowohl für (a) als auch für (b) der N-Träger während der gesamten Dauer der Vorbereitung in fester Phase vorliegt, unlöslich in der Reaktionsumgebung ist und biologisch abbaubar ist.

2. Verfahren nach Anspruch 1, **dadurch gekennzeichnet, dass** die Bildung des Konjugats von Hyaluronsäure, Palmitoyl-Hyaluronat oder Formyl-Hyaluronat 2 bis 48 Stunden lang bei einer im Bereich von 20 °C bis 40 °C liegenden Temperatur, vorzugsweise 24 Stunden lang bei einer im Bereich von 22 °C bis 25 °C liegenden Temperatur, erfolgt.

3. Verfahren nach einem der vorhergehenden Ansprüche, **dadurch gekennzeichnet, dass** das aprotische polare Lösungsmittel aus der Gruppe ausgewählt ist, die *N,N-*Dimethylformamid, Dimethylsulfoxid, *N*-Methyl-2-Pyrrolidon, Acetonitril, Dichlormethan, Tetrahydrofuran und 1,4-Dioxan umfasst, und/oder dass die basische Umgebung, in der die terminale N-schützende Gruppe gespaltet wird, aus der Gruppe ausgewählt ist, die 20% Piperidin in DMF, 2% DBU in DMF und 30% Tert-Butylamin in DMF umfasst.

4. Verfahren nach Anspruch 1, **dadurch gekennzeichnet, dass** die medizinische Substanz A an den N-Träger durch Veresterung gebunden ist, wobei die medizinische Substanz A eine Aminosäure oder ein Peptid mit einer gebundenen terminalen N-schützenden Gruppe und einer Carboxylgruppe ist, wobei die medizinische Substanz A zunächst einer Aktivierung der Carboxylgruppe mittels eines Kondensierungsreagenz in einem aprotischen polaren Lösungsmittel unter Bildung eines reaktiven Zwischenprodukts unterzogen wird, welches Zwischenprodukt anschliessend mit einem N-Träger beim Vorhandensein einer organischen Base und eines Katalysators reagieren gelassen wird, und anschliessend die an den Träger gebundene terminale N-schützende Gruppe der medizinische Substanz A in einer basischen Umgebung gespaltet wird, um ein Konjugat nach der allgemeinen Formel X zu bilden, in der S -O-C(=O)- oder ein linearer Linker ist, welcher von einem Peptid dargestellt ist, das von hydrophobischen Aminosäuren Xaa gebildet ist.

5. Verfahren nach Anspruch 4, **dadurch gekennzeichnet, dass** das Kondensierungsreagenz aus der Gruppe ausgewählt ist, die N,N'-DiisopropylCarbodiimid, N,N'-Dicyklohexyl-Carbodiimid, 1-[Bis(dimethylamino) Methylen]-1H-1,2,3-Triazol[4,5-b]-Pyridinium 3-Oxid-Hexafluorophosphat, 1-Ethyl-3-(3-DimethylAminopropyl)-Carbodiimid, Anhydrid der Propylphosphonsäure, 2-(1H-Benzotriazol-1-yl)-1,1,3,3-Tetramethyl-Uronium-Hexafluorophosphat, Chlorameisensäureethylester, Benzotriazol-1-yl-Oxy-Tris(dimethylamino)-Phosphonium-Hexafluorophosphat, O-(Benzotriazol-1-yl)-N,N,N',N'-Tetramethyl-Uronium-Tetrafluoroborat, 4-Triethylamino-Dicyklohexyl-Carbodiimid-p-Toluen-Sulfonat, 4-Nitrophenol, N-Hydroxysuccinimid, 2,4,5-Trichlorophenol, 2,3,4,5,6-Pentafluorophenol sowie 2,3,4,5,6-Pentachlorophenol umfasst.

6. Verfahren nach Anspruch 4, **dadurch gekennzeichnet, dass** die organische Base aus der Gruppe ausgewählt ist, die aus Triethylamin, Pyridin, Morpholin, *N*-MethylMorpholin, *N,N'*-Diisopropyl-Ethylamin und Imidazol besteht, und/oder der Katalysator aus der Gruppe ausgewählt ist, die Ethyl(hydroxyimino)-Kyanoacetat, Hydroxybenzotriazol, 1-Hydroxy-7-Azabenzotriazol oder *N,N*-Dimethylaminopyridin umfasst.

7. Verfahren nach Anspruch 4, **dadurch gekennzeichnet, dass** das Konjugat ferner einer wiederholten Reaktion mit einem reaktiven Zwischenprodukt unterzogen wird, wobei das identisch mit oder unterschiedlich von dem im vorherigen Schritt eingesetzten reaktiven Zwischenprodukt sein kann.

8. Verfahren nach Anspruch 4, **dadurch gekennzeichnet, dass** das Kondensierungsreagenz *N,N'*-Diisopropyl-Carbodiimid ist, das aprotische polare Lösungsmittel *N,N*-Dimethylformamid ist, als der Katalysator die Kombination von Ethyl(hydroxyimino)-Kyanoacetat und *N,N*-Dimethylaminopyridin eingesetzt wird und die basische Umgebung 20% Piperidin in N,N-Dimethylformamid ist.

9. Verfahren nach einem der Ansprüche 1 bis 8, **dadurch gekennzeichnet, dass** der Anteil der medizinischen Substanz A 1 bis 5 Äquivalenten entspricht, bezogen auf ein Dimer von Hyaluronsäure oder Palmitoyl-Hyaluronat oder Formyl-Hyaluronat.

10. Verfahren nach einem der Ansprüche 4 bis 8, **dadurch gekennzeichnet, dass** der Anteil des Kondensierungsreagenz 0,1 bis 5 Äquivalenten entspricht, bezogen auf ein Dimer von Hyaluronsäure oder Palmitoyl-Hyaluronat oder Formyl-Hyaluronat, und/oder der Anteil der organischen Basis bis 10 Äquivalenten entspricht, bezogen auf ein Dimer von Hyaluronsäure oder Palmitoyl-Hyaluronat oder Formyl-Hyaluronat, und/oder der Anteil des Katalysators 0,1 bis 5 Äquivalenten entspricht, bezogen auf ein Dimer von Hyaluronsäure oder Palmitoyl-Hyaluronat oder Formyl-Hyaluronat, wobei vorzugsweise 3 Äquivalente von Ethyl(hydroxyimino)-Kyanoacetat und 0,3 Äquivalente von *N,N*-Dimethylaminopyridin eingesetzt werden.

11. Verfahren nach Anspruch 4, **dadurch gekennzeichnet, dass** der Anteil der medizinischen Substanz A 3 Äquivalenten entspricht, der Anteil des Kondensierungsreagenz 3 Äquivalenten entspricht und der Anteil des Katalysators ebenfalls 3 Äquivalenten entspricht, bezogen auf ein Dimer von Hyaluronsäure oder Palmitoyl-Hyaluronat oder Formyl-Hyaluronat.

12. Verfahren nach Anspruch 4, **dadurch gekennzeichnet, dass** der Anteil der medizinischen Substanz A 1 Äquivalent entspricht, der Anteil des Kondensierungsreagenz 1 Äquivalent entspricht und der Anteil des Katalysators ebenfalls 1 Äquivalent entspricht, bezogen auf ein Dimer von Hyaluronsäure oder Palmitoyl-Hyaluronat oder Formyl-Hyaluronat.

13. Verfahren nach einem der Ansprüche 1 bis 3 **dadurch gekennzeichnet, dass** die medizinische Substanz A an den N-Träger durch eine in einem aprotischen polaren Lösungsmittel und beim Vorhandensein eines reduzierenden Reagenz stattfindende reduzierende Aminierungsreaktion gebunden ist und anschliessend die an den Träger gebundene terminale N-schützende Gruppe der medizinische Substanz A in einer basischen Umgebung gespaltet wird, um ein Konjugat nach der allgemeinen Formel X zu bilden, in der S -NH- oder ein linearer Linker ist, welcher von einem Peptid dargestellt ist, das von hydrophobischen Aminosäuren Xaa gebildet ist.

14. Verfahren nach Anspruch 13, **dadurch gekennzeichnet, dass** das Konjugat ferner einer wiederholten Reaktion mit der medizinischen Substanz A unterzogen wird, die identisch mit oder unterschiedlich von der im vorherigen Schritt eingesetzten medizinischen Substanz A sein kann.

15. Verfahren nach Anspruch 13, **dadurch gekennzeichnet, dass** als das aprotische polare Lösungsmittel DMF, als das reduzierende Reagenz picBH₃ und als die basische Umgebung eine 20%-ige Lösung von Piperidin im DMF eingesetzt wird.

16. Verfahren nach Anspruch 1, **dadurch gekennzeichnet, dass** die medizinische Substanz A an den Träger mittels eines linearen Linkers Xaa-Ahx-Ahx-Xaa gebunden ist.

## Revendications

1. Procédé de préparation d'une préparation médical à base de l'hyaluronane et/ou palmitoyl hyaluronane et/ou formyl hyaluronane selon la formule générale (X) :
**A-S-N** **(X)**
représentée par la formule : 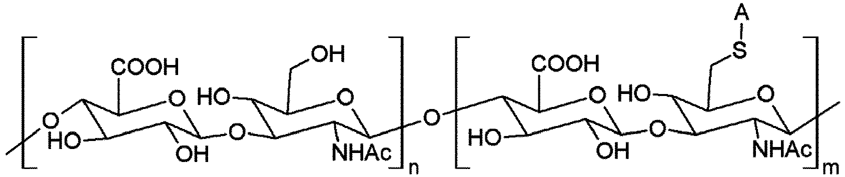 où
A est un peptide ou un acide aminé,
S est -O-C(=O)-, -NH-, ou une liaison linéaire qui est un peptide, formé d'acides aminés hydrophobiques Xaa,
m est de 10 à 1250,
n est de 100 à 12500,
**caractérisé en ce que**
(a) la préparation poursuit comme une réaction d'estérification selon le Régime 1 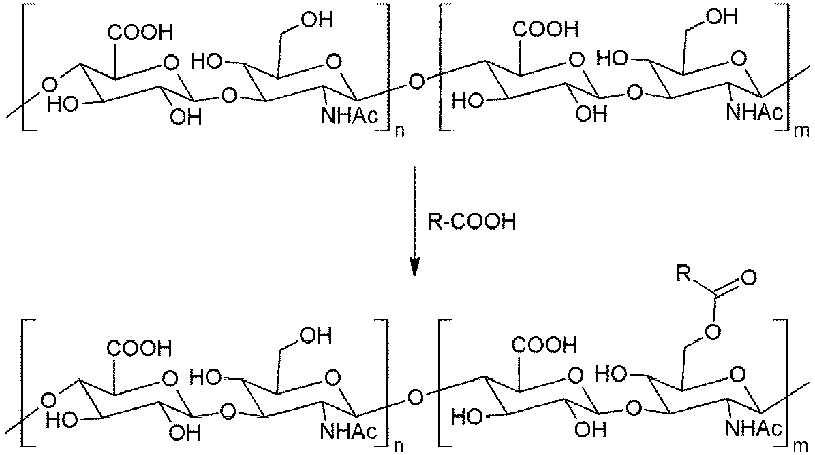 où R est un peptide ou un acide aminé,
où la substance médicale A comprenant un groupe terminale N-protecteur et un groupe carboxyle, la substance médicale A étant sélectée dans le groupe consistant en acides aminés et en peptides, est au premier soumise à l'activation du groupe carboxyle par un agent de condensation dans un solvant polaire aprotique pour former un intermédiaire réactif qui est ensuite mis à réagir avec un support N, le support N étant l'acide hyaluronique, palmitoyle hyaluronane ou formyle hyaluronane et ayant le poids moléculaire dans le plage de 1.5x10⁴ à 2.5x10⁶ g.mol⁻¹, et le support N étant en forme d'un fibre continue, fil, textile, couche mince, fibre discontinue et/ou textile non-tissé, dans la présence d'une base organique et d'un catalyseur, puis le groupe terminale N-protecteur de la substance médicale A attaché au support est découpé dans un environnement basique de manière à former le conjugué selon la formule générale X, où S est -O-C(=O)-, ou une liaison linéaire qui est un peptide, formé par acides aminés hydrophobiques Xaa.
ou
(b) la préparation poursuit comme une réaction d'amination réductive selon le Régime 2 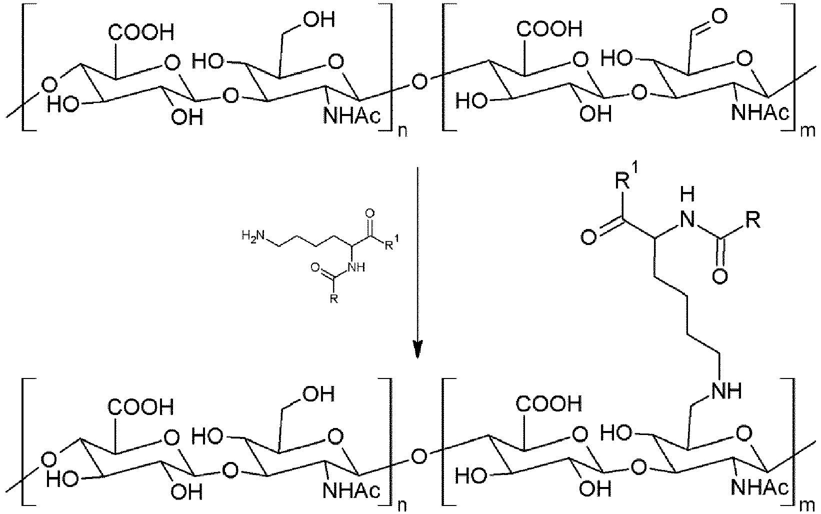 où R est un acide aminé ou un peptide, et R¹ est un amide ou un methylester, où la substance médicale A comprenant un groupe terminale N-protecteur et étant sélecté dans le groupe consistant en acides aminés et peptides est mis à réagir avec un support N dans un solvant polaire aprotique et dans la présence d'un agent de réduction, le support N étant acide hyaluronique et/ou palmitoyle hyaluronane et/ou formyle hyaluronane et ayant le poids moléculaire dans le plage de 1.5x10⁴ à 2.5x10⁶ g.mol⁻¹, le support N étant en forme d'un fibre continue, fil, textile, couche mince, fibre discontinue et/ou textile non-tissé, puis le groupe terminale N-protecteur de la substance médicale A attaché au support est découpé dans un environnement basique de manière à former le conjugué selon la formule générale X, où S est -NH-, ou une liaison linéaire qui est un peptide formé par acides aminés hydrophobiques Xaa, où pour (a) et (b) le support N est dans une phase solide pendant la préparation entière, est insoluble dans l'environnement de réaction et biodégradable.

2. Le procédé selon la revendication 1 **caractérisé en ce que** la formation du conjugué d'acide hyaluronique et palmitoyle hyaluronane et formyle hyaluronane est effectuée à la température de 20 °C à 40 °C pendant 2 à 48 heures, avantageusement à la température de 22 °C à 25 °C pendant 24 heures.

3. Le procédé selon l'une quelconque des revendications précédentes **caractérisé en ce que** le solvant polaire aprotique es sélectionné dans le groupe consistant en *N*-*N*-dimethyl formamide, dimethyl sulphoxide, *N*-methyl-2-pyrrolidone, acetonitrile, dichlormethane, tetrahydrofuran, 1,4-dioxane et/ou l'environnement basique, dans lequel le groupe terminal N-protecteur est découpé, est sélectionné dans le groupe consistant en 20% de piperidine en DMF, 2% DBU en DMF, 30% tert-butylamine en DMF.

4. Le procédé selon la revendication 1, **caractérisée en ce que** la substance médicale A est attaché au support N au moyen de l'estérification, où la substance médicale A est un acide aminé ou un peptide comprenant un groupe terminal N-protecteur et un groupe carboxyle, où la substance médicale A est au premier soumise à l'activation du groupe carboxyle par un agent de condensation dans un solvant polaire aprotique pour former un intermédiaire réactif qui est ensuite mis à réagir avec le support N dans la présence d'une base organique et d'un catalyseur, puis le groupe terminal N-protecteur de la substance médicale A attaché au support est séparé dans uns environnement basique de manière à former le conjugué selon la formule générale X, où S est -O-C(=O)-, ou une liaison linéaire qui est un peptide formé d'acides aminés hydrophobiques Xaa.

5. Le procédé selon la revendication 4 **caractérisé en ce que** l'agent de condensation est sélectionné dans le groupe de N,N'-diisopropyl carbodiimide, N,N'-dicyclohexyl carbodiimide, 1-[bis(dimethylamino)methylen]-1H-1,2,3-triazolo[4,5-b] pyridinium 3-oxide hexyfluoro phosphate, 1-ethyl-3-(3-dimethyl aminopropyl) carbodiimide, anhydride d'acide propylphosphonique, 2-(1H-benzotriazole-1-yl)-1,1,3,3-tetramethyl uronium hexafluoro phosphate, ethyl choloroformiate, benzotriazole-1-yl-oxy-tris(dimethylamino) phosphonium hexafluoro phosphate, O-(benzotriazole-1-yl)-N,N,N',N'-tetramethyl uronium tetrafluoroborate, 4-triethylamino-dicyclohexal carbodiimide p-toluen sulfonate, 4-nitrophenol, N-hydroxy succinimide, 2,4,5-trichlorophenol, 2,3,4,5,6-pentafluoro phenol, 2,3,4,5,6-pentachloro phenol.

6. Le procédé selon la revendication 4 **caractérisé en ce que** la base organique est sélectionné dans le groupe consistant en triethylamine, pyridine, morpholine, *N*-methyl morpholine, *N,N'-*diisopropyl ethylamine, imidazole et/ou le catalyseur est sélectionné dans le groupe consistant en ethyl(hydroxyimino) cyanoacétate, hydroxybenzo triazole, 1-hydroxy-7-azabenzotriazole, ou *N,N*-dimethylamino pyridine.

7. Le procédé selon la revendication 4 **caractérisé en ce que** le conjugué est davantage à plusieurs reprises mis à réagir avec l'intermédiaire réactif qui peut être le même ou différent de l'intermédiaire réactif de l'étape précédente.

8. Le procédé selon la revendication 4 **caractérisé en ce que** l'agent de condensation est *N*,*N'*-diisopropyl carbodiimide, le solvant polaire aprotique est *N,N*-dimethyl formamide, le catalyseur est la combinaison d'ethyl (hydroxyimino) cyanoacétate et *N,N*-dimethylamino pyridine et l'environnement basique est 20% de piperidine dans du *N,N*-dimethyl formamide.

9. Le procédé selon l'une quelconque des revendications 1 à 8 **caractérisé en ce que** la quantité de la substance médicale A correspond à 1 à 5 équivalents relativement à un dimer d'acide hyaluronique ou de palmitoyle hyaluronane ou de formyle hyaluronane.

10. Le procédé selon l'une quelconque des revendications 4 à 8 **caractérisé en ce que** la quantité de l'agent de condensation correspond à 0.1 à 5 équivalents relativement à un dimer d'acide hyaluronique ou de palmitoyle hyaluronane ou de formyle hyaluronane et/ou la quantité de la base organique correspond à jusqu'à 10 équivalents, relativement à un dimer d'acide hyaluronique ou de palmitoyle hyaluronane ou de formyle hyaluronane et/ou la quantité de catalyseur correspond à 0.1 à 5 équivalents, relativement à un dimer d'acide hyaluronique ou de palmitoyle hyaluronane ou de formyle hyaluronane, avantageusement 3 équivalents d'ethyl(hydroxyimino) cyanoacétate et 0.3 équivalents de *N,N*-dimethylamino pyridine sont utilisés.

11. Le procédé selon la revendication 4 **caractérisé en ce que** la quantité de substance médical A correspond à 3 équivalents, la quantité d'agent de condensation correspond à 3 équivalents et la quantité de catalyseur correspond à 3 équivalents relativement à un dimer d'acide hyaluronique ou de palmitoyle hyaluronane ou de formyle hyaluronane.

12. Le procédé selon la revendication 4 **caractérisé en ce que** la quantité de substance médicale A correspond à 1 équivalent, la quantité d'agent de condensation correspond à 1 équivalent et la quantité de catalyseur correspond à 1 équivalent relativement à un dimer d'acide hyaluronique ou de palmitoyle hyaluronane ou de formyle hyaluronane.

13. Le procédé de préparation selon l'une quelconque des revendications 1 à 3, **caractérisé en ce que** la substance médicale A attachée au support N au moyen de l'amination réductive dans un solvant polaire aprotique et dans la présence de l'agent de réduction et ensuite le groupe terminal N-protecteur de la substance médicale A attachée au support est découpé dans un environnement basique de manière à former le conjugué selon la formule générale X, où S est -NH-, ou une liaison linéaire qui est un peptide formé d'acides aminés hydrophobiques Xaa.

14. Le procédé selon la revendication 13 **caractérisé en ce que** le conjugué est davantage à plusieurs reprises mis à réagir avec la substance médicale A qui peut être la même ou différent de la substance médicale A de l'étape précédente.

15. Le procédé de préparation selon la revendication 13 **caractérisé en ce que** le solvant polaire aprotique est DMF, l'agent de réduction est picBH₃ et l'environnement basique est 20% solution de piperidine en DMF.

16. Le procédé selon la revendication 1 **caractérisé en ce que** la substance médicale A est attachée au support via une liaison linéaire Xaa-Ahx-Ahx-Xaa.


### SEQUENCE LISTING

<110> Contipro a.s.
<120> Medical preparation with a carrier based on hyaluronan and/or derivatives thereof, method of prepration and use thereof
<130> D4126-PCT/Lu
<150> CZPV 2016-826
   <151> 2016-12-22
<160> 15
<170> BiSSAP 1.3.6
<210> 1
   <211> 9
   <212> PRT
   <213> Artificial Sequence
<220>
   <221> DISULFID
   <222> 1..6
   <223> disulfide bridge
<220>
   <223> Oxytocin
<400> 1
   Cys Tyr Ile Gln Asn Cys Pro Leu Gly
   1               5
<210> 2
   <211> 9
   <212> PRT
   <213> Artificial Sequence
<220>
   <221> MOD_RES
   <222> 1
   <223> Mpr SULFATATION
<220>
   <221> DISULFID
   <222> 1..6
   <223> disulfide bridge
<220>
   <223> Desmopressin
<400> 2
   Xaa Tyr Phe Gln Asn Cys Pro Arg Gly
   1               5
<210> 3
   <211> 6
   <212> PRT
   <213> Artificial Sequence
<220>
   <223> Dalargin
<400> 3
   Tyr Ala Gly Phe Leu Arg
   1               5
<210> 4
   <211> 9
   <212> PRT
   <213> Artificial Sequence
<220>
   <221> MOD_RES
   <222> 1
   <223> pGlu PYRROLIDONE CARBOXYLIC ACID
<220>
   <223> Lecirelin
<220>
   <221> MOD_RES
   <222> 9
   <223> NHEt
<400> 4
   Glu His Trp Ser Tyr Leu Leu Arg Pro
   1               5
<210> 5
   <211> 9
   <212> PRT
   <213> Artificial Sequence
<220>
   <221> MOD_RES
   <222> 1
   <223> pGlu PYRROLIDONE CARBOXYLIC ACID
<220>
   <223> Fertitrelin
<220>
   <221> MOD_RES
   <222> 9
   <223> NHEt
<400> 5
   Glu His Trp Ser Tyr Gly Leu Arg Pro
   1               5
<210> 6
   <211> 12
   <212> PRT
   <213> Artificial Sequence
<220>
   <223> Terlipressin
<220>
   <221> DISULFID
   <222> 4..9
   <223> disulfide bridge
<400> 6
   Gly Gly Gly Cys Tyr Phe Gln Asn Cys Pro Lys Gly
   1               5                   10
<210> 7
   <211> 9
   <212> PRT
   <213> Artificial Sequence
<220>
   <221> DISULFID
   <222> 1..6
   <223> disulfide bridge
<220>
   <223> Lysipressin
<400> 7
   Cys Tyr Phe Gln Asn Cys Pro Lys Gly
   1 5
<210> 8
   <211> 7
   <212> PRT
   <213> Artificial Sequence
<220>
   <221> MOD_RES
   <222> 1
   <223> Ac ACETYLATION
<220>
   <223> Effect on adhesive properties
<220>
   <221> MOD_RES
   <222> 7
   <223> Nle
<400> 8
   Arg Gly Asp Gly Gly Gly Xaa
   1 5
<210> 9
   <211> 7
   <212> PRT
   <213> Artificial Sequence
<220>
   <221> MOD_RES
   <222> 1
   <223> Fmoc
<220>
   <223> Effect on adhesive properties
<220>
   <221> MOD_RES
   <222> 7
   <223> Nle
<400> 9
   Arg Gly Asp Gly Gly Gly Xaa
   1 5
<210> 10
   <211> 6
   <212> PRT
   <213> Artificial Sequence
<220>
   <221> MOD_RES
   <222> 1
   <223> Ac ACETYLATION
<220>
   <223> Effect on adhesive properties
<400> 10
   Gly Gly Gly Arg Gly Asp
   1               5
<210> 11
   <211> 6
   <212> PRT
   <213> Artificial Sequence
<220>
   <221> MOD_RES
   <222> 1
   <223> Fmoc
<220>
   <223> Effect on adhesive properties
<400> 11
   Gly Gly Gly Arg Gly Asp
   1               5
<210> 12
   <211> 6
   <212> PRT
   <213> Artificial Sequence
<220>
   <221> MOD_RES
   <222> 1
   <223> Ac ACETYLATION
<220>
   <223> Effect on adhesive properties
<220>
   <221> MOD_RES
   <222> 4
   <223> Ahx
<220>
   <221> MOD_RES
   <222> 5
   <223> Ahx
<220>
   <221> MOD_RES
   <222> 6
   <223> Nle
<400> 12
   Arg Gly Asp Xaa Xaa Xaa
   1 5
<210> 13
   <211> 6
   <212> PRT
   <213> Artificial Sequence
<220>
   <221> MOD_RES
   <222> 1
   <223> Fmoc
<220>
   <223> Effect on adhesive properties
<220>
   <221> MOD_RES
   <222> 4
   <223> Ahx
<220>
   <221> MOD_RES
   <222> 5
   <223> Ahx
<220>
   <221> MOD_RES
   <222> 6
   <223> Nle
<400> 13
   Arg Gly Asp Xaa Xaa Xaa
   1 5
<210> 14
   <211> 5
   <212> PRT
   <213> Artificial Sequence
<220>
   <221> MOD_RES
   <222> 1
   <223> Ahx
<220>
   <221> MOD_RES
   <222> 1
   <223> Ac ACETYLATION
<220>
   <223> Effect on adhesive properties
<220>
   <221> MOD_RES
   <222> 2
   <223> Ahx
<400> 14
   Xaa Xaa Arg Gly Asp
   1               5
<210> 15
   <211> 5
   <212> PRT
   <213> Artificial Sequence
<220>
   <221> MOD_RES
   <222> 1
   <223> Ahx
<220>
   <221> MOD_RES
   <222> 1
   <223> Fmoc
<220>
   <223> Effect on adhesive properties
<220>
   <221> MOD_RES
   <222> 2
   <223> Ahx
<400> 15
   Xaa Xaa Arg Gly Asp
   1               5